(19) Europäisches Patentamt
European Patent Office
Office européen des brevets

(11) **EP 1 776 148 B1**

(12) **EUROPEAN PATENT SPECIFICATION**

(45) Date of publication and mention
of the grant of the patent:
**06.03.2019 Bulletin 2019/10**

(21) Application number: **05772220.9**

(22) Date of filing: **12.08.2005**

(51) Int Cl.:
*A61L 27/24* (2006.01)     *A61L 27/54* (2006.01)
*A61F 2/14* (2006.01)      *G02B 1/00* (2006.01)

(86) International application number:
**PCT/CA2005/001240**

(87) International publication number:
**WO 2006/015490 (16.02.2006 Gazette 2006/07)**

(54) **OPHTHALMIC DEVICES AND RELATED METHODS AND COMPOSITIONS**

OPHTHALMISCHE VORRICHTUNGEN UND VERWANDTE VERFAHREN UND
ZUSAMMENSETZUNGEN

DISPOSITIFS OPHTALMIQUES ET PROCÉDÉS ET COMPOSITIONS ASSOCIÉS

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **13.08.2004 US 601270 P**

(43) Date of publication of application:
**25.04.2007 Bulletin 2007/17**

(73) Proprietors:
• **Ottawa Hospital Research Institute
Ottawa, Ontario K1H 8L6 (CA)**
• **NATIONAL RESEARCH COUNCIL OF CANADA
Ottawa, Ontario K1A OR6 (CA)**

(72) Inventors:
• **GRIFFITH, May
Carp, Ontario K0A 1L0 (CA)**
• **CARLSSON, David J.
Ottawa, Ontario K1J 7E6 (CA)**
• **LI, Fengfu
Ottawa, Ontario K1J 1J1 (CA)**
• **LIU, Yuwen
Ottawa, Ontario K1V 6L7 (CA)**
• **RAFAT, Merhdad
Gatineau, Québec J9H 7K7 (CA)**

(74) Representative: **J A Kemp
14 South Square
Gray's Inn
London WC1R 5JJ (GB)**

(56) References cited:
WO-A1-2004/014969     CA-A1- 2 134 744
CA-A1- 2 286 718       US-A- 4 223 984
US-A- 4 581 030        US-A- 4 703 108
US-A1- 2003 018 123

• LI F. ET AL: 'Cellular and nerve regeneration
within a bioprosthetic extracellular matrix for
corneal implantation' PROC. NATL. ACAD. SCI.
vol. 100, no. 26, 23 December 2003, pages 15346
- 15351, XP008112136
• LI F. ET AL: 'Recruitment of multiple cell lines by
collagen-synthetic copolymer matrices in
corneal regeneration' BIOMATERIALS vol. 26, no.
16, June 2005, pages 3093 - 3104, XP025280615
• CARLSSON D.J. ET AL: 'Bioengineered corneas:
how close are we?' CURR. OPIN. OPHTHALMOL.
vol. 14, no. 4, August 2003, pages 192 - 197,
XP008113757
• LESKUL M. ET AL: 'CxGELSIX: a novel
preparation of type VI collagen with possible use
as a biomaterial' CORNEA vol. 19, no. 2, March
2000, pages 194 - 203, XP008113773
• DOILLON C.HJ. ET AL: 'A collagen-based
scaffold for a tissue engineered human cornea:
physical and physiological properties' INT. J.
ARTIF. ORGANS. vol. 26, no. 8, August 2003,
pages 764 - 773, XP008098193

**Description**

## BACKGROUND OF THE INVENTION

1. Field of the invention

The present invention relates to devices, methods, and compositions for enhancing the vision of an individual or for treating a traumatic injury of an eye or an ophthalmic disease or disorder in an individual. In particular, the invention relates to corneal onlays, corneal inlays, and corneal implants that are made of a material that provides one or more benefits to the individual.

2. Description of related art

WO2004/014969 A1 discloses a bio-synthetic matrix comprising a co-polymer comprising one or more N-alkyl or N,N-dialkyl substituted acrylamide co-monomer, one or more hydrophilic co-monomer and one or more acryl- or methacryl- carboxylic acid co-monomer derivatised to contain a pendant moiety capable of crosslinking bioactive molecules, such as peptides (e.g. YIGSR). The matrix is prepared by dispersing the synthetic co-polymer and a bio-polymer (e.g. collagen of bovine origin) in an aqueous medium and allowing the synthetic co-polymer and the bio-polymer to crosslink. The amount of the bio-polymer is 0.3-50% by weight. In order to preserve triple helix structure of polymers such as collagen and to prevent fibrillogenisis and/ or opacification of the hydrogel, the crosslinking reaction should be conducted in aqueous media with close control of the pH and temperature. The matrix can be used as artificial cornea.
U.S. Pat. No. 5,713,957 discloses corneal onlays which comprise a non-biodegradable non-hydrogel ocularly biocompatible material, and having a porosity sufficient to allow passage through the onlay of tissue fluid components having a molecular fluid weight greater than 10,000 Dalton.
U.S. Pat. No. 5,716,633 discloses a collagen/PHEMA-hydrogel for promoting epithelial cell growth and regeneration of the stroma. The collagen-hydrogel may be provided as an optical lens to be affixed to Bowman's membrane, which is effective to promote and support epithelial cell growth or attachment of the corneal epithelium over the anterior surface of the lens. The collagen-hydrogel is a hydrogel polymer formed by the free radical polymerization of a hydrophilic monomer solution gelled and cross-linked in the presence of an aqueous stock solution of collagen to form a three dimensional polymeric meshwork for anchoring collagen. The final concentration of collagen in the onlay is from about 0.3% to about 0.5% (wt/wt).
U.S. Pat. No. 5,836,313 discloses methods for forming implantable composite keratoprostheses. The methods provide keratoprostheses designed to provide a suitable substrate for corneal epithelial cell growth. The keratoprostheses are formed by placing corneal tissue in a mold having a corneal implant shape and cross-linking a polymeric solution to chemically bond a biocompatible hydrogel having a thickness between approximately 50 and 100 microns to the corneal tissue to form the keratoprosthesis. Or, a polymer solution is placed between the corneal tissue and a pre-formed hydrogel and then polymerized so that the polymer solution couples to both the hydrogel and the corneal tissue
U.S. Pat. No. 6,454,800 discloses a corneal onlay or corneal implant that comprises a surface with a plurality of surface indentations that supports the attachment and growth of tissue cells.
U.S. Pat. No. 6,689,165 discloses a synthetic device for cornea augmentation and replacement that increases corneal epithelium cell adhesion and migration using tethered corneal enhancer agents.
Some problems associated with existing collagen-based materials are that the collagen based materials are not optically clear, which may be due to the formation of, or conversion into, a fibrous based material, which results in undesirable light scattering.
Thus there remains a need for materials which are biocompatible, ophthalmically acceptable, and are suitable for placement in an eye to enhance an individual's vision.

## SUMMARY OF THE INVENTION

An ophthalmic device comprises a body including a composition effective in facilitating nerve growth through or over the body when the device is placed in an eye of an individual. In certain embodiments, the device is a vision enhancing ophthalmic device. In alternative embodiments, the device is a therapeutic ophthalmic device. The present vision enhancing devices can be understood to be devices that are structured to correct one or more refractive errors. In other words, the present devices can be understood to be refractive error correcting devices. The body of the present devices can be formed to have an optical power.
The present invention provides an optically clear biosynthetic, implantable corneal device, comprising cross-linked collagen, wherein the cross-linked collagen is obtainable by a process of cross-linking collagen polymers using 1-ethyl-3-(3-dimethylaminopropyl) carbodiimide (EDC) and N-hydroxysuccinimide (NHS), and wherein the composition comprises an amount of collagen between 5% and 50% by weight or volume.

In one embodiment, collagen is the sole polymer.

In another embodiment, collagen is the sole water-swellable polymer.

In a further embodiment, collagen is the sole lens-forming polymer.

Preferably, the amount of collagen is

(a) at least 6% by weight or volume;
(b) at least 10% by weight or volume;
(c) between 10% and 30% by weight or volume; or
(d) between 10% and 24% by weight or volume.

It is also preferred that the cross-linked collagen comprises:

(a) one type of collagen; or
(b) two or more types of collagen.

It is additionally preferred that the device further comprises a cell growth enhancer agent. Typically, the cell growth enhancer agent is a peptide or is selected from neurotrophic factors, nerve growth factors, and epidermal growth factors. Preferably, the peptide has an amino acid sequence of RGD, YIGSR, or IKVAV. It is also preferred that the cell growth enhancer agent is distributed substantially throughout the device.

In one embodiment, the collagen:

(a) is the sole water-swellable polymer of the device; and/or
(b) was cross-linked at an acidic pH.

Preferably, in such an embodiment the acidic pH is between 5.0 and 5.5.

Preferably, according to the invention, the cross-linked collagen comprises:

(a) atelocollagen, type I collagen, type III collagen, or a combination thereof;
(b) recombinant collagen; or
(c) collagen isolated from an animal.

It is also preferred that the cross-linked collagen is produced by mixing together the collagen polymers and the EDC/NHS cross-linker at an acidic PH while preventing surges in pH.

It is further preferred that the mixing of polymers and crosslinker agent is at high shear.

It is additionally preferred that the device further comprises chondroitin sulfate, N,O-carboxymethylchitosan, hyaluronic acid, hyaluronic acid aldehyde or alginate.

The composition of the present invention is optically clear and comprises an amount of collagen between 5% (w/v or w/w) and 50% (w/v or w/w). As used herein, the amount of collagen and/or other components of the compositions and devices will be understood to be either w/w or w/v percentages without departing from the spirit of the invention. In additional embodiments, the material may comprise an amount of collagen between about 10% and about 30%. The material comprises an amount of cross-linked collagen between about 5% and 50%, wherein the collagen is cross-linked using 1-ethyl-3-(3-dimethylaminopropyl)carbodiimide (EDC; CAS # 1892-57-5) and N-hydroxysuccinimide. The material may comprise a first collagen polymer cross-linked to a second collagen polymer. In certain embodiments, the ophthalmic devices disclosed herein are manufactured without glutaraldehyde. For example, the ophthalmic devices do not utilize glutaraldehyde as a cross-linker in the manufacture thereof. Glutaraldehyde may not be desirable or preferred to use as a cross-linking agent due to handling and safety requirements for glutaraldehyde and/or the present compositions and devices. In certain embodiments, the ophthalmic devices are manufactured without cytotoxic components, or in other words, are manufactured using components having a reduced cytotoxicity.

The foregoing device may be a corneal onlay, a corneal inlay, or a full-thickness corneal implant, such as a device configured to replace an individual's natural cornea. The present devices are transparent, and may be produced from compositions which are transparent before the compositions are formed into the devices.

The material of the foregoing device may also comprise one or more cell growth enhancer agents or one or more additional biopolymers.

Also provided is a method of making a composition of the invention, comprising:

combining collagen polymers using 1-ethyl-3-(3-dimethylaminopropyl)carbodiimide and N-hydroxysuccinimide cross-linker agents at an acidic pH; and

curing the resultant combination to form the composition comprising cross-linked collagen.

In such a method, it is preferable that:

(a) the combining step comprises mixing the collagen polymers and the cross-linker agent in a system configured to produce high shear forces on the resultant combination; and
(b) the combining occurs at a temperature between 0°C and 5°C.

Preferably, the method further comprising adding a cell growth enhancer agent to the combination.

A method of making an ophthalmic device, such as a refractive error correcting device, in accordance with the disclosure herein comprises cross-linking collagen polymers using 1-ethyl-3-(3-dimethylaminopropyl)carbodiimide and N-hydroxysuccinimide (EDC and NHS). The cross-linking occurs at an acidic pH, such as a pH of about 5.0 to about 5.5. The method may also comprise one or more steps of adding a cell growth enhancer agent to the cross-linked composition. The method comprises placing the composition in a mold, and allowing the composition to cure to form an ophthalmic device.

Also provided is a device according to the invention for use in the treatment of an ophthalmic disease, disorder or injury in a subject in need thereof. Preferably, an eye of the subject having said ophthalmic disease, disorder or injury is contacted with said ophthalmic device.

Further provided is a device according to the invention for use in facilitating nerve growth on and/or into the device.

Any feature or combination of features described herein are included within the scope of the present invention provided that the features included in any such combination are not mutually inconsistent as will be apparent from the context, this specification, and the knowledge of one of ordinary skill in the art. In addition, any feature or combination of features may be specifically excluded from any embodiment of the present invention.

Additional advantages and aspects of the present invention are apparent in the following detailed description, drawings, examples, and claims.

## BRIEF DESCRIPTION OF THE DRAWINGS

FIG. 1 is an illustration of a sectional view of a T-piece adapter of a system for producing the present compositions and devices.

FIG. 2 is an illustration of a sectional view of a female Luer adapter of a system for producing the present compositions and devices.

FIG. 3 is a plan view of the T-adapter of FIG. 1 with a septum and two syringes coupled thereto to produce the present compositions and devices.

FIG. 4 is a graph of cell count as a function of time for a human recombinant hydrogel material designated F1.

FIG. 5 is a graph of cell count as a function of time for a human recombinant hydrogel material designated F3.

FIG. 6 is a graph of cell count as a function of time for a human recombinant hydrogel material designated F6.

FIG. 7 is a photograph of a human recombinant hydrogel material designated F3 located in a rat.

FIG. 8 is an illustration of one embodiment of the present refractive error correcting ophthalmic devices.

FIG. 8A is an illustration of a lens edge configuration of one embodiment of the present onlays.

FIG. 9 is a graph of swell ratio as a function of EDC to $NH_2$ mole ratio.

FIG. 10 is a graph of tensile strength as a function of EDC to $NH_2$ mole ratio.

FIG. 11 provides graphs of tensile strength as a function of collagen concentration (left panel) and swelling ratio as a function of collagen concentration (right panel).

FIG. 12 provides graphs of heat flow as a function of temperature for compositions having different EDC to $NH_2$ mole ratios (left panel), and heat flow as a function of temperature for compositions having different CSC concentrations (right panel).

FIG. 13 is a graph of neurite length as a function of chondroitin sulfate to collagen dry weight ratio.

## DETAILED DESCRIPTION

A typical human eye has a lens and an iris. The posterior chamber is located posterior to the iris and the anterior chamber is located anterior to iris. The eye has a cornea that consists of five layers, as discussed herein. One of the layers, the corneal epithelium, lines the anterior exterior surface of cornea. The corneal epithelium is a stratified squamous epithelium that extends laterally to the limbus.

The five layers of the cornea include the corneal epithelium, the Bowman's membrane, the stroma, Descemet's membrane, and the endothelium. The corneal epithelium usually is about 5-6 cell layers thick (approximately 50 micrometers thick), and generally regenerates when the cornea is injured. The corneal epithelium provides a relatively smooth refractive surface and helps prevent infection of the eye. The corneal stroma is a laminated structure of collagen which contains cells, such as fibroblasts and keratocytes, dispersed therein. The stroma constitutes about 90% of the corneal thickness. The anterior portion of the stroma, which underlies the epithelium, is acellular and is known as Bowman's membrane. Bowman's membrane is located between the epithelium and the stroma and is believed to protect the cornea from injury. The corneal endothelium typically is a monolayer of low cuboidal or squamous cells that dehydrates the cornea by removing water from the cornea. An adult human cornea is typically about 500 $\mu$m (0.5 mm) thick and is typically devoid of blood vessels.

Ophthalmic devices have been invented which provide one or more benefits to an individual, such as a person, who desires their vision to be enhanced or improved or who is in need of treatment of a disease, disorder or traumatic injury of the eye. The devices described herein may be configured as corneal onlays, corneal inlays, or full-thickness corneal implants. The present devices may enhance vision of an individual who has reduced vision or provide vision to an individual who has no vision. The devices described herein specifically exclude intraocular lenses.

As used herein, "optically clear" refers to at least 85% transmission of white light. In certain embodiments, "optically clear" refers to optical clarity that is equivalent to that of a healthy cornea, for example, having greater than 90% transmission of white light and less than 3% scatter.

As used herein, a "corneal onlay" is an ophthalmic implant or device configured, such as sized and shaped, to be located between the epithelium or an epithelial cell layer and Bowman's membrane of an individual's eye, such as a human's or animal's eye. In comparison, a contact lens is configured to be located over the epithelium of an eye. A corneal onlay may thus rest entirely over the Bowman's membrane, or it may include one or more portions that extend into Bowman's membrane. Such portions constitute a minor portion of the device, such as less than 50% of the area or volume of the device.

As used herein, a "corneal inlay" is a device or implant configured to be placed in the stroma of an eye. Corneal inlays may be placed in the stroma by forming a flap or a pocket in the stroma. Corneal inlays are placed below the Bowman's membrane of an eye.

As used herein, a "full-thickness corneal implant", refers to a device that is configured to replace all or part of an unhealthy cornea of an eye located anterior to the aqueous humor of the eye.

The present ophthalmic devices have a reduced cytotoxicity or are non-cytotoxic and provide one or more benefits to an individual in which the device is placed. For example, the devices provide one or more of the following: (i) a desired refractive index, (ii) a desired optical clarity (for visible light, optical transmission and light scattering equal to or better than those of healthy human cornea material of comparable thickness), (iii) a desired optical power, such as a vision enhancing optical power, (iv) enhanced comfort, (v) enhanced corneal and epithelial health, and (vi) therapeutic benefit, for example, in the treatment of a disease, disorder or traumatic injury of an eye. The present ophthalmic devices are transparent or are formed of a transparent material. Some examples of such devices include devices which are optically clear.

The foregoing benefits, as well as others, may be obtained by forming the device of a material that is (i) shapeable, such as moldable, to form a matrix with an acceptable optical power, (ii) optically clear or visually transparent, and (iii) effective in facilitating nerve growth through and/or over the device. When the device is a corneal onlay, the device is effective in facilitating re-epithelialization over the anterior surface of the device.

The device is formed of a material that has sufficient mechanical or structural properties to survive handling, implantation, which may include suturing, and post-installation wear and tear. The device provides or permits sufficient nutrient and gas exchange to promote a healthy eye. The devices that are produced in molds, such as corneal onlays, are formed of a material which can be molded to the appropriate size and shape, including edge gradient and vision corrective curvature, as discussed herein.

In one embodiment of the present invention, a vision enhancing ophthalmic device comprises a body including a material that is effective in facilitating nerve growth through the body when the device is placed in an eye of an individual. By facilitating nerve growth through the body, corneas of individuals receiving the device or devices maintain their touch sensitivity. The body is formed to have an optical power. Thus, the body may be understood to be a lens body. As discussed herein, the device may be configured, such as sized and shaped, to be a corneal onlay, a corneal inlay, or a full-thickness corneal implant. In certain embodiments, the present refractive error correcting devices may not have an optical power. For example, refractive error correcting devices in accordance with the present disclosure may be understood to be blanks that can be placed between a patient's corneal epithelium and Bowman's membrane, or in the patient's corneal stroma.

For corneal onlays, the material from which the onlay is produced provides for or permits gas and nutrient, such as glucose, exchange between the Bowman's membrane and epithelium to maintain a viable, fully functioning epithelium. Other nutrients include factors or agents to promote or enhance the survival, growth, and differentiation of cells, such as epithelial cells. The exchange should be comparable to or better than that of a healthy human cornea. The permeability of the material to nutrients and/or drugs may be monitored using conventional techniques. In addition, the movement of the nutrients and/or drugs through the material should not cause the optical properties of the material to change. The onlays or lenticules are fully biocompatible, allow rapid epithelial adhesion to the onlay, and permit restoration of nerve innervation and sensitivity, for example touch sensitivity.

The present ophthalmic devices may comprise an extracellular matrix (ECM) component. In certain devices, the material of the body comprises, consists essentially of, or consists of, collagen. The collagen may be cross-linked, for example by using EDC/NHS in the manufacture of the devices. The amount of collagen provided in the present hydrogel devices is greater than what is currently used in other ophthalmic devices. The amount of collagen provided in the present devices is between 5% and 50% (w/w) or (w/v), as discussed herein. In certain embodiments of the present devices, the amount of collagen is greater than about 6% (w/w). Or, the material may comprise an amount of collagen between about 10% (w/w) and about 30% (w/w). As understood by persons of ordinary skill in the art, about 15 wt% of a hydrated human cornea is collagen (Maurice D M: The Cornea and Sclera, pp489-600. The Eye, Vol I, Second ed., Ed. H Davson. Academic Press, New York, 1969). Thus, the present devices include an amount of collagen that is greater than existing ophthalmic devices and is much more similar to the amount of collagen present in human corneas. In addition, the amount and type of collagen provided in the present devices is effective in providing a desired refractive index, a desired optical clarity, shapability, permitting handling, implantation, and suturing of the device in the eye, and post-installation wear and tear.

The remaining portion of the ophthalmic device, such as the non-collagen based portion, may be a liquid, such as water or saline, or may also include one or more additional polymers, such as biopolymers and the like. For example, an ophthalmic device which comprises about 24% (w/w) collagen, as disclosed herein, may include about 76% (w/w) of a liquid, such as water or saline. In other words, in a hydrated state, the ophthalmic device may have a collagen component that is 24% of the weight of the hydrated ophthalmic device. As another example, an ophthalmic device may comprise a collagen component that is 24% of the weight of the hydrated device, and a second polymeric component that is 6% of the weight of the hydrated device, and 70% of the weight is a liquid.

As understood by persons of ordinary skill in the art, in the unhydrated state, the amount of collagen in the device may be a greater percentage than in the hydrated state.

Collagen comprises three polypeptide chains and is helical in structure. As used herein, the term "collagen polymer" is intended to refer to a triple helical collagen molecule. Collagen is a rod-like molecule with a length and diameter of about 300 nm and about 1.5 nm, respectively. A collagen molecule has an amino acid sequence called a "telopeptide" on both its N- and C-terminals, which include most of the antigenicity of collagen. Atelocollagen is obtained by pepsin digestion [DeLustro et al., J Biomed Mater Res. 1986 Jan;20(1):109-20] and is free from telopeptides, indicating that it has low immunogenicity [Stenzel et al., Annu Rev Biophys Bioeng. 1974;3(0):231-53].

The collagen used in the above-identified devices may be obtained or derived from any suitable source of collagen including animal, yeast, and bacterial sources. For example, the collagen may be human collagen, bovine collagen, porcine collagen, avian collagen, murine collagen, equine collagen, among others, or the collagen may be recombinant collagen. Recombinant collagen in the present devices can include one or more structural or physical features that are not present in collagen obtained from normal animal sources since recombinant collagen is obtained from bacteria, ,yeast, plants or transgenic animals. For example, recombinant human collagen can include different glycosylation components that may not be present in animal-derived and processed collagen. In addition, recombinant collagen can have different degrees of cross-linking relative to animal derived collagen, which can be of variable composition. Variation in cross-linking degrees in animal derived collagens can result in inconsistencies and variable chemical and physical properties of the collagen that may not be desirable. As well as being of tightly controlled purity, recombinant human collagen is not associated with viral and/or prion contamination, which may be associated with animal derived collagen. Collagen useful in the present devices is publicly available or can be synthesized using conventional techniques. For example, recombinant collagen may be obtained from Fibrogen (from mutigene yeast bioreactor culture) or Pharming (Netherlands) (from the milk of transgenic cows or rabbits), or recombinant collagen may be prepared and obtained using the methods disclosed in PCT Publication No. WO 93/07889 or WO 94/16570. In certain devices, the collagen may be type I collagen. The devices may also be made of atelocollagen (e.g., collagen without telopeptides). In certain embodiments, the collagen is a non-denatured type of collagen. Atelocollagen may be obtained from companies such as Koken Japan (Supplier A, as used herein), in which bovine collagen is available as 3.5% (w/v) in a neutral composition, 3.0% (w/v) in an acidic composition, 10% (w/v) in an acidic composition, and in which porcine collagen is available as 3.0% (w/v) in an acidic composition, or as acidic, freeze dried porcine collagen powder. Acidic, freeze dried porcine collagen powder may also be obtained from Nippon Ham (Japan) (Supplier B, as used herein). Becton Dickinson (Supplier C, as used herein) provides 0.3% acidic and 10% acidic collagen compositions.

Of the several collagen types, atelocollagen I provides for ease of solution, handling and final device clarity. This collagen (bovine, porcine or recombinant, either in neutral or acidic solution, or as an acidic, freeze-dried powder) is available from several companies, as described above. Freeze dried, acidic porcine collagen dissolves readily to give homogenous (non-opalescent) aqueous solutions in cold water at up to 33% (w/v) concentration by stirring at 4°C. The pH of these clear collagen compositions, such as solutions, is about 3 (Supplier B) or about 5 (Supplier A). Commercial acidic collagen compositions as low as 0.3% (w/v), can be concentrated by vacuum evaporation with stirring at 0° - 4°C to give clear solutions of up to about 10% (w/v) final collagen concentration, which may then be used in the manufacture of the present devices.

Relatively tough or strong ophthalmic devices may be obtained using collagen type I that has not been denatured (i.e. lost all or a substantial portion of its triple helix conformation to become gelatin) during isolation and purification.

Differential scanning calorimetry (DSC) is a useful tool to determine the quality of solutions of suppliers' collagen based on their triple helix content (Table 1). For near perfect triple helix content, the DSC enthalpy of denaturing ($\Delta H_{denature}$) is in the 65 - 70 J/g range (based on the dry collagen weight) . From DSC data, $\Delta H_{denature}$ results indicate that solutions from commercial, acidic, freeze dried, porcine collagens and some of the commercial, bovine collagen solutions are in the fully, triple helix form.

Collagen solutions with low triple helix content ($\Delta H_{denature}$ < 5 J/g, supplier C, Table 1) have relatively low viscosity and give weak gels as compared to compositions or solutions of the same concentration from collagens with close to 100% triple helix content. Collagen compositions (solutions) with $\Delta H_{denature}$ > about 60 J/g were found to make acceptable ophthalmic devices.

**Table 1. Enthalpy of denaturing of collagen solutions**

| Commercial collagen samples | Composition | $\Delta H_{denature}$ (J/g of dry collagen) |
|---|---|---|
| Koken (Japan), Supplier A | 10% bovine solution | 65.3 |
| Koken (Japan), Supplier A | 10% bovine collagen solution, concentrated from 3% acidic | 67.5 |
| Koken (Japan), Supplier A | 5% bovine collagen solution, concentrated from 3.0% acidic | 66.4 |
| Koken (Japan), Supplier A | 3.5% bovine neutral solution | 68.1 |
| Koken (Japan), Supplier A | 3.5% bovine neutral solution, after heat denaturing | 24.4 |
| Koken (Japan), Supplier A | 3.0% porcine collagen solution | 72.0 |
| Koken (Japan), Supplier A | 5% Solution from freeze dried, porcine collagen (acidic) | 68.1 |
| Nippon Ham, Supplier B | 10% Solution from freeze dried, porcine collagen (very acidic) | 63.4 |
| Becton Dickinson, Supplier C | 5% bovine solution concentrated from 0.3% | 59.4 |
| Becton Dickinson, Supplier C | "10%" bovine solution | 4.8 |
| FibroGen recombinant human collagen | 10% solution, concentrated from 0.3 wt/wt% | 67.7 |

In certain embodiments, including those described above, the material of the body may comprise a collagen polymers that are cross-linked. Or, stated differently, the material of the body may comprise two or more cross-linked collagen polymers. For example, the material of the body may comprise a first collagen polymer, a second collagen polymer, and a third collagen polymer. Other materials may comprise more than three collagen polymers. The cross-linked polymers may be understood to be a collagen component of the ophthalmic device.

Thus, a vision enhancing ophthalmic device in accordance with the present invention comprises a collagen component having an amount of collagen between 5% (w/w) and 50% (w/w) and being formed to have an optical power. As discussed herein, in certain embodiments, the amount of collagen is greater than about 6% (w/w). For example, the amount of collagen is between about 10% (w/w) and about 30% (w/w). For example, the amount of collagen may be between about 10% (w/w) and about 24% (w/w). In certain devices, the collagen is the sole water-swellable (e.g., hydrogel) polymer of the device. In other devices, the collagen may be the sole device or lens forming polymer. For example, the device may comprise 100% collagen in a dry state. As discussed above, in certain devices, the collagen may be cross-linked, or at least partially cross-linked, using EDC/NHS, for example.

The collagen polymers used in the manufacture of the compositions and devices of the present invention may be from the same collagen source or from different collagen sources. Or, stated differently, a single type of collagen, for example an atelocollagen type I (which contains a plurality of collagen polymer chains), is processed in a manner effective to permit the collagen polymers to cross-link to each other. In one embodiment, the collagen polymers are recombinant collagen. In other embodiments, both the collagen polymers are derived from the same animal source. Individual collagen polymers in a single composition may have different molecular weights.

It may be understood that the present refractive error correcting devices comprise cross-linked recombinant collagen. The amount of collagen present in such devices is greater than that found in other collagen based refractive error correcting devices that have been previously disclosed. Such devices may be formed to have an optical power.

The devices disclosed herein are transparent. For example, the devices should be optically clear. For example, the device should provide minimal light scattering (comparable to or better than healthy, human cornea tissue) when the device is placed in an eye of an individual. In addition, the device disclosed herein has a refractive index. In certain embodiments, the refractive index is between about 1.34 and about 1.37. For example, the refractive index may be between 1.341 and 1.349. When the device is configured as a corneal onlay or corneal inlay, the device is configured to be placed in a healthy eye of an individual, as compared to an eye in which the cornea is damaged or diseased where a full-thickness corneal implant may be needed. In certain embodiments of the present devices, the devices do not have a yellow tinge or a yellow color. For example, the devices may be designed to reduce or eliminate a yellow tinge or a yellow color that may be associated with some collagen containing compositions.

The present device has an anterior surface and a posterior surface. Thus, the body or the collagen component of the device may have an anterior surface and a posterior surface. The anterior and posterior surfaces are generally opposing surfaces. The anterior surface of the device refers to the surface that is oriented away from the retina when the device is placed in an eye, and the posterior surface is oriented toward the retina when the device is placed in an eye. When the device is a corneal onlay, the posterior surface will be adjacent to, and may contact, Bowman's membrane, and the anterior surface will be adjacent to and may contact the corneal epithelium. When the device is a corneal inlay, the anterior surface will be adjacent or oriented towards the Bowman's membrane, and the posterior surface will be in the stroma oriented towards the retina of the eye. When the device is a full-thickness corneal implant, the anterior surface is oriented toward the corneal epithelium, and the posterior surface is adjacent and may be in contact with the corneal endothelium.

The present device may comprise no additional surface modification, or the device may comprise a surface modification that affects cell growth and/or differentiation on either or both of the anterior and posterior surfaces. For example, a corneal onlay may include no surface modification that affects cell growth on the anterior or posterior surface. As used herein, "cell growth" refers to an expansion of a cell or a population of cells. Thus, cell growth refers to the physical growth of an individual cell, such as an increase in surface area, volume, and the like, proliferation of a cell or cells, such as the division of cells, and the migration of cells, in some cases to form a stratified multilayer as found on a healthy human cornea. Cell growth refers to growth of nerve cells, such as the extension of one or more neuronal processes over, under or through the device, and to the growth or migration or proliferation of epithelial cells or endothelial cells over a surface of the device. As used herein, "cell differentiation" refers to the morphological, biochemical and physiological changes that a single or population of totipotent, multipotent or immature precursor cells (including stem cells) undergoes to achieve its final phenotype. In certain embodiments of the present devices, epithelial cells grow over the corneal onlay and are tightly coupled thereto, for example directly attached to the onlay, specifically the anterior surface of the onlay.

In certain corneal onlays, the body or collagen component includes a posterior surface modification effective in reducing epithelial cell growth under the onlay when the onlay is placed in an eye of an individual. In addition, or alternatively, corneal onlays may include a body or collagen component that includes an anterior surface modification effective in promoting epithelial cell growth, including migration, over the anterior surface of the onlay when the onlay is placed in an eye of an individual. Relatedly, full-thickness corneal implants may include a body or collagen component that includes a posterior surface modification effective in reducing endothelial cell growth over the posterior surface of the full-thickness corneal implants when the implant is placed in an eye. Full-thickness corneal implants may include no anterior surface modifications.

Examples of surface modifications that may reduce cell growth include providing a plasma polymerized fluorinated monomer film, such as $CF_4$ or $C_3F_8$ on one or both of the anterior and posterior surfaces, providing a low free surface energy on one or both of the surfaces, and/or by making one or both of the surfaces hydrophilic. The surfaces may be made hydrophilic by providing an alginate coating over the surface or surfaces.

The devices may include one or more cell growth enhancer agents that facilitate cell growth on or through the device. In certain embodiments, the cell growth enhancer agent comprises a peptide. For example, the cell growth enhancer agent may be a peptide having an amino acid sequence that includes RGD, YIGSR, or IKVAV. Collagen I itself is a rich source of RGD sequences. In certain embodiments, the cell growth enhancer agent is a neurotrophic factor or its bioactive or neurotrophic portions of the molecule. For example, the neurotrophic factor may be nerve growth factor (NGF), an epidermal growth factor (EGF or HB-EGF) or basic fibroblast growth factor (bFGF or FGF-2). The cell growth enhancer agent may be integrally formed with the collagen component or body of the device, or in other words, the cell growth enhancer agent may be provided substantially throughout the device. In comparison, some ophthalmic devices only include peptides provided on one surface of the device.

In certain embodiments, the collagen-based ophthalmic devices comprise a collagen component which was processed at an acidic pH in the manufacture of the device. An acidic pH is particularly useful when the collagen component comprises a first collagen polymer cross-linked to a second collagen polymer. The acidic pH used in the manufacture of such devices is typically less than about 6.0, for example, the pH may be between about 5.0 and about 5.5. By maintaining an acidic pH and by preventing or reducing pH surges during pH adjustments, fibrillogenesis of the collagen is reduced. In addition, by maintaining the pH above about 5.0, the collagen does not degrade as quickly as if the pH were less than 5.0.

The collagen polymers may be cross-linked using any small or polymeric, collagen-reactive agent or molecule. The cross-linking chemistry may employ conventional methods which are routine to persons of ordinary skill in the art or novel reagents. By cross-linking the collagen polymers, the devices maintain their optical clarity and are able to withstand biodegradation.

In certain embodiments, the collagen polymers are cross-linked using 1-ethyl-3-(3-dimethylaminopropyl)carbodiimide (EDC; CAS #1892-57-5) and N-hydroxysuccinimide (NHS). In other words, a crosslinking agent used in the manufacture of the device is EDC/NHS. The collagen polymers and the EDC/NHS cross-linker are mixed together at an acidic pH while preventing surges in pH. After sufficient mixing, portions of the mixed composition are placed in a mould, and are allowed to cure in the mould to form an ophthalmic device. One advantage of utilizing the water-soluble EDC/NHS chemistry to crosslink collagen and CSC is that it results in a zero length (amide) bond. This reduces the possibility of grafted toxic substances leaching out into tissues. In addition, unreacted reagents and by-products from the EDC/NHS reaction are water-soluble and thus can be removed easily after gel formation.

In certain embodiments, the collagen polymers are cross-linked using a cross-linker or cross-linking agent that has a reduced cytotoxicity. Such cross-linkers preferably do not irritate or cause a negative reaction when the ophthalmic devices are placed in an eye of an individual. In some embodiments, the cross-linker is a cross-linker other than glutaraldehyde. Although glutaraldehyde may be a useful cross-linker in certain embodiments, glutaraldehyde may not be preferred due to handling and safety requirements.

In additional embodiments, the process may further comprise using at least one of the following components: poly(N-isopropylacrylamide-co-acrylic acid), a chondroitin sulfate, a keratan sulfate, a dermatan sulphate, elastin, chitosan, N,O-carboxymethylchitosan, hyaluronic acid, hyaluronic acid aldehyde, and alginate, which may be mixed with the collagen compositions. Thus, the ophthalmic devices may comprise a collagen component, such as a matrix of cross-linked collagen polymers, and one or more non-collagen polymers, including biopolymers. The non-collagen polymers may be cross-linked together and/or may be cross-linked to the collagen polymers to form a network or matrix of cross-linked polymers.

In certain embodiments, the compositions are mixed together through relatively narrow channels or passageways to induce a high shear between the different compositions. In one embodiment, the compositions are mixed using a syringe-based system. The mixing depends on the syringe pumping through narrow channels to induce high shear between the viscous collagen solution and the reagents. The channel diameter is chosen to accommodate the viscosity and burst strength of the syringes or other similar devices. For high viscosities (e.g., 20-30% (w/v) collagen solutions), small volume syringes with small diameter syringe plungers are used because higher pressures can be obtained by hand. The mixing occurs at an acidic pH, such as between about 5.0 and about 5.5 and at a reduced temperature, such as between about 0°C and about 5°C.

Compared to other ophthalmic devices, the present devices are manufactured without the use of living cells. Thus, the present inventors have invented new methods of manufacturing compositions and ophthalmic devices with relatively high, and nearly physiological concentrations, of collagen without using living corneal cells. In addition, the present devices are substantially or entirely free of a synthetic dendrimer component, which has been used to increase the cross-reactivity of collagen in other devices.

Additional nerve-friendly materials may be used in the manufacture of the present devices. Such materials may be manufactured using the methods disclosed herein and tested for nerve-friendliness, such as nerve growth, using conventional methods which are routine to persons of ordinary skill in the art, such as cell culture systems, and the like. For example, the materials can be tested and identified using the methods disclosed in WO 2004/015090, filed August 11, 2003.

The devices disclosed herein are configured, such as sized and shaped, to be placed in an eye around the corneal region of the eye. When the device is a corneal onlay, the onlay may have a diameter from about 4 mm to about 12 mm, such as about 6 mm. The onlay may also have an edge thickness less than about 30 $\mu$m, for example, between about 10 um and about 30 $\mu$m. The onlay may also have a center thickness of about 70 $\mu$m.

Onlay shaped moulds may be manufactured from polypropylene and may have diameters of 4 mm, 6 mm, 8 mm, or 12 mm. The moulds should be relatively stiff (e.g., non-flexing during closure), and transparent for allowing charge visualization. The moulds are configured to provide a fine taper (e.g., about 10 $\mu$m) onlay edge, or a somewhat steeper (e.g., about 30 $\mu$m) onlay edge.

Corneal implant moulds (either full-thickness or partial-thickness) may have diameters of about 12 mm. The transplant moulds are shaped with a desired curvature and thickness of the cornea. If necessary, the ophthalmic device (e.g., hydrogel) can be trephined out as needed for the transplantation procedure.

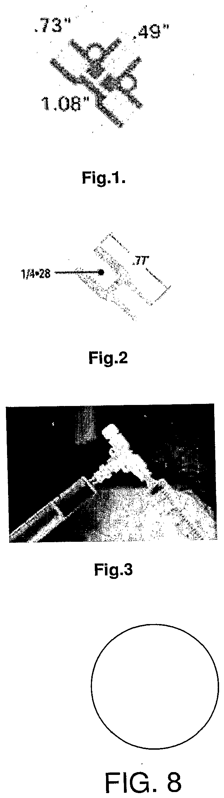

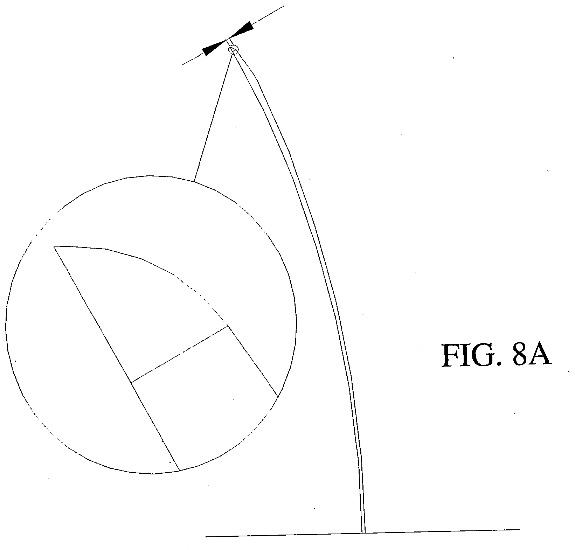

An example of one of the present refractive error correcting devices is illustrated in FIG. 8 and FIG. 8A.

The corneal onlays disclosed herein may also be configured to correct for one or more wavefront aberrations of an individual's eye. A description of wavefront technology and the measurements of wavefront aberrations is provided in U.S. Pat. No. 6,086,204 (Magnate) and WO 2004/028356 (Altmann). The corneal onlays may be shaped to correct for a wavefront aberration by shaping the mould in a desired configuration which permits the onlay to assume the corrective shape. Methods of using wavefront aberration measurements in corneal onlays is disclosed in U.S. Application No. 60/573,657, filed May 20, 2004. The onlays may also be ablated to correct for a wavefront aberration. For example, the onlays may be ablated using a laser or laser-like device, a lathe, and other suitable lens shaping devices.

The corneal onlays disclosed herein may also include a plurality of different zones. For example, the corneal onlay may include an optic zone and a peripheral zone. Typically, the optic zone is bounded by the peripheral zone, or in other words, the optic zone is generally centrally located about an optical axis, such as a central optical axis, of the onlay and the peripheral zone is disposed between an edge of the optic zone and the peripheral edge of the corneal onlay. Additional zones and onlay configurations may be provided with the onlay depending on the particular visual deficiency experienced by the patient.

In addition, the present corneal onlays may have junctionless zones, such as two or more zones that do not have a visually or optically detectable junction. The zones of the onlays may be smooth and continuous, and the onlays may be optically optimized to correct not only refractive errors, but also other optical aberrations of the eye and/or the optical device independently or in combination with correcting refractive errors. As understood by persons skilled in the art, a corneal onlay may be structured to correct visual deficiencies including, and not limited to, myopia, hyperopia, astigmatism, and presbyopia. The onlay may enhance or improve visual deficiencies by either optical means or physical means imposed on the stroma of the eye, or a combination thereof. Thus, the corneal onlay may be a monofocal lens or a multifocal lens, including, without limitation, a bifocal lens.

In addition, or alternatively, the corneal onlay may be a toric lens. For example, the onlay may include a toric region which may be effective when placed on an eye with an astigmatism to correct or reduce the effects of the astigmatism. The onlay may include a toric region located on the posterior surface of the onlay, or the onlay may include a toric region located on the anterior surface. Advantageously, toric onlays may be used without requiring a ballast to maintain proper orientation of the onlay on the eye since the onlay may be held in a relatively fixed position by the epithelium of the appliance. However, a ballast may be provided if desired. In certain embodiments, the onlay may include a ballast, such as a prism, or it may include one or more thinned regions, such as one or more inferior and/or superior thin zones. In onlays configured to correct presbyobia, the onlay may include one or more designs, such as concentric, aspheric (either with positive and/or negative spherical aberration), diffractive, and/or multi-zone refractive.

The present invention also encompasses compositions, such as synthetic or non-naturally occurring compositions. The compositions may be fully or partially synthetic. For example, the invention relates to compositions that are optically clear. Such compositions may be used in the manufacture of one or more ophthalmic devices disclosed herein. Alternatively, the compositions could be used in non-ophthalmic settings as non-ophthalmic compositions, or can be used in ophthalmic settings and not provide a refractive error correction. In another embodiment, a composition, in accordance with the present disclosure, comprises an amount of collagen that is between 5% and 50% (w/w) in a hydrated stated and is optically clear. As discussed herein, the composition may comprise an amount of collagen 5.0% and 30% (w/w) in a hydrated state. In certain embodiments, the composition may comprise about 6% (w/w) of collagen. In other embodiments, the composition may comprise an amount of collagen between about 10% (w/w) and about 24% (w/w). The composition may comprise an amount of cross-linked collagen that is between 5% and 50% (w/w) in a hydrated state, wherein the collagen is cross-linked using EDC/NHS.

The present compositions may comprise two or more collagen polymers. In certain embodiments, the compositions comprise a first collagen polymer cross-linked to a second collagen polymer as described above. The compositions may be substantially or completely free of cytotoxic agents, such as glutaraldehyde.

The ophthalmic devices disclosed herein may be placed in an eye using any suitable methodology or technique.

For example, corneal onlays may be placed over the Bowman's membrane of an eye by removing or separating a portion of the epithelium from the Bowman's membrane. In certain situations, an amount of alcohol, such as ethanol, may be applied to the corneal epithelium to delaminate the epithelium from the eye. The alcohol may be at a concentration of about 10% to about 60%, for example about 20% or about 50%. Warming the ethanol to about 37°C (e.g., body temperature) may be effective to enhance the epithelial removal. This deepithelial technique is similar to the LASEK technique that is currently being practiced.

In other situations, a corneal onlay may be placed over Bowman's membrane by placing the onlay under an epithelial flap or in an epithelial pocket. Such flaps and pockets may be made using a cutting instrument, a blunt dissection tool, and the like. Examples of methods of placing a corneal onlay in an eye are disclosed in U.S. Application No.

10/661,400, filed September 12, 2003, and U.S. Application No. 60/573,657, filed May 20, 2004.

Corneal inlays may be placed in an eye by forming a intrastromal pocket or a corneal flap, and placing the inlay in the pocket or under the flap.

Full-thickness corneal implants may be placed in an eye by removing a damaged or diseased portion of a cornea and placing the corneal implant in or near the region of the removed portion of the cornea.

The ophthalmic devices disclosed herein may be placed in an eye using forceps, or any other suitable inserter, such as those described in U.S. Application No. 10/661,400, filed September 12, 2003, and U.S. Application No. 60/573,657, filed May 20, 2004.

To facilitate placement of the ophthalmic device in the eye, the device may include a visualization component. The visualization component can be any suitable feature that permits the device to be easily seen while being inserted or placed in an eye. For example, the visualization component may include one or more markings, which may also help with the rotational position of the device, or the visualization component may include a dye, such as a biocompatible or non-cytotoxic dye, or a tinting agent.

Additional details regarding the present ophthalmic devices and related methods of manufacturing and using the devices are provided in the examples below, which are provided by way of illustration, and do not limit the invention.

**EXAMPLES**

**Example 1**

Preparation of collagen-based corneal onlays.

Typically, 0.5 mL - 2.0 mL of a collagen solution in an aqueous buffer was mixed with 0.01 mL - 0.50 mL of a cross-linker agent in the aqueous buffer at about 0°C without air bubble entrapment. In some compositions, a second biopolymer other than collagen was added to the composition.

To mix the compositions, syringes containing the compositions were connected to a Tefzel Tee-piece (Uptight Fittings), forming a micro-manifold that allowed thorough mixing of the viscous collagen solution and/or controlled neutralization without surges in pH. A pH surge often led to irreversible fibrillogenesis of the collagen to give opaque matrices.

More specifically, a first Luer adaptor was used to retain a septum, cut to size to fit tightly into the bottom of the Tee's thread hole. Septa were cut to size from Restek Corporations's, "Ice Blue" 17 mm general purpose 22397 septa. A first syringe with a buffer solution, such as MES (2-[N-morpholino]ethanesulfonic acid) buffer was locked into the second Luer adaptor and any air bubbles were pushed out with the buffer solution. A collagen solution was placed in a second syringe which was then connected to the third Luer adaptor of the Tefzel Tee-piece (as shown in FIG. 1) fitted with three Luer adaptors (FIG. 2). The full assembly is shown in FIG. 3.

The collagen solution was completely mixed with the MES buffer solution by repeated pumping between the first and second syringes through the Tee so that flow through the narrow bore channels (for example between about 0.5 mm to about 0.25 mm) in the Tee piece strongly sheared the liquid. The pH was adjusted to 5.0 - 5.5. The collagen/buffer mixture was then mixed with the EDC and NHS solution (EDC:NHS at 1:1 molar equivalents ratio) at 0°C-4°C by directing the compositions through the manifold using another syringe.

Aliquots of each substantially homogeneous solution were immediately dispensed into onlay moulds and cured first at room temperature for 5 - 24 hours, such as 15 hours, and then at 37°C for 15 - 24 hours, in 100% humidity environments at both temperatures.

Each final onlay sample was carefully separated from its mould after immersion in phosphate buffered saline (PBS) for 2 hours.

In some cases, these gels were immersed in an aqueous solution of a second reactive biopolymer to give further cross-linking and to add new biological factors.

Finally, the cross-linked onlay hydrogels were immersed in PBS solution (0.5% in PBS, containing 1% chloroform) at 20°C to terminate any reactive residues and to extract out reaction byproducts. These sterile, equilibrium hydrated onlays were thoroughly rinsed in PBS before all testing.

For gels prepared from some collagen/EDC-NHS chemistries, at the higher collagen concentrations (10% and above), gels were first soaked in pH 9.1 buffer to terminate any residual reactivity and give adequate extraction of reaction products before storage in chloroform-saturated PBS. This basic extraction removed epithelial toxicity problems for these samples. For many stoichiometries, soaking in chloroform saturated PBS, followed by removal of chloroform residues gave sterile, non-cytotoxic gels.

### Example 2

Ophthalmic devices with cell growth enhancer agents

Cell growth enhancer agents, such as the pentapeptide (YIGSR, the active unit in the laminin macromolecule) alone or in combination with synergistic peptides such as one containing, IKVAV, synergistic IGF, and substance P peptides that promote epithelial health, EGF, NGF, FGF or portions of these molecules can be incorporated into any of the collagen/EDC-NHS, cross-linked devices, including those with a second, EDC-NHS reactive bio-polymer. For YIGSR, the coupling of this cell growth enhancer agent may be achieved via the reactivity of the free amine terminal group of a tyrosine residue on the agent. Extensive extraction after gellation may be used to remove any unbound cell growth enhancer agent.

Specific formulation details of ophthalmic devices are provided in Examples 3-13 and in Table 2, below.

### Example 3

An ophthalmic device was made as described in Example 1 using 1-ethyl-3-(3-dimethylaminopropyl)carbodiimide (EDC) with N-hydroxysuccinimide (NHS) + collagens at pH 5.5 in MES buffer, at 0° - 4°C raised to 21°C for 15 h, then 15 h at 37°C. EDC:NHS = 1:1 molar equivalents ratio.

### Example 4

An ophthalmic device was made as described in Example 1 using COP + EDC-NHS + collagens at pH 5.5 in MES buffer, at 0° 4°C raised to 21°C for 15 h, then 15 h at 37°C. EDC:NHS = 1:1 molar equivalents ratio. [COP, copolymer, poly(N-isopropylacrylamide-co-acrylic acid) was prepared by free-radical polymerization of NiPAAm and AAc in 1,4-dioxane with 2,2'-azobis-isobutyronitrile initiator under nitrogen at 70 °C] .

### Example 5

An ophthalmic device was made as described in Example 1 using EDC-NHS + chondroitin sulfate C (ChS) + collagens at pH 5.5 in MES buffer, at 0° - 4°C raised to 21°C for 15 h, then 15 h at 37°C. EDC:NHS = 1:1 molar equivalents ratio.

### Example 6

An ophthalmic device was made as described in Example 1 using collagens + EDC-NHS + N,O-carboxymethylchitosan (CMC) at pH 5.5 in MES buffer, at 0° - 4°C, raised to 21°C for 15 h, then 15 h at 37°C. EDC:NHS = 1:1 molar equivalents ratio.

### Example 7

An ophthalmic device was made as described in Example 1 using collagens + EDC-NHS + N,O-carboxymethylchitosan (CMC) at pH 5.5 in MES buffer, at 0° - 4°C, raised to 21°C for 2 h, then + second cross-linking when gel immersed in chitosan (1% aqueous solution, 5000 Da) in PBS for 4 h. Finally 15 h at 37°C. EDC:NHS = 1:1 molar equivalents ratio.

### Example 8

An ophthalmic device was made as described in Example 1 using collagens + EDC-NHS + hyaluronic acid (HA) and at pH 5.5 in MES buffer, at 0° - 4°C, raised to 21°C for 15 h, then 15 h at 37°C. EDC:NHS = 1:1 molar equivalents ratio.

### Example 9

An ophthalmic device was made as described in Example 1 using collagens + EDC-NHS + Chondroitin sulfate (ChS) + hyaluronic acid (HA) at pH 5.5 in MES buffer, at 0° - 4°C, raised to 21°C for 15 h, then raised to 37°C for 15 h. EDC:NHS = 1:1 molar equivalents ratio.

**Reference Example 10**

An ophthalmic device was made as described in Example 1 using collagens + hyaluronic acid aldehyde (HA-CHO) + sodium cyanoborohydride at pH 7 - 8 in PBS at 0° - 4°C, raised to 21°C for 15 h, then 15 h at 37°C. HA-CHO was prepared from HA (0.1 g) by oxidative cleavage with sodium periodate (0.05 g) for 2 h at 21°C. The aqueous solution was dialysed against water for 2 days.

**Example 11**

An ophthalmic device was made as described in Example 1 using collagens + EDC-NHS + alginate at pH 5.5 in MES buffer, at 0° - 4°C, raised to 21°C for 15 h, then 15 h at 37°C. EDC:NHS = 1:1 molar equivalents ratio.

**Reference Example 12**

An ophthalmic device was made as described in Example 1 using glutaraldehyde ("Glut", diluted to 1% in water)+ collagens at pH 5.5 in MES buffer at 0° - 4°C raised to 21°C for 2 h, then + second cross-linking when gel immersed in chitosan (1% aqueous solution, 5000 Da) in PBS for 4 h. Gels in their moulds were raised to 37°C for 15 h before removal under PBS.

**Example 13**

An ophthalmic device was made as described in Example 1 using collagens + EDC-NHS + chitosan at pH 5.5 in MES buffer, at 0° - 4°C, raised to 21°C for 15 h, then 15 h at 37°C. EDC:NHS - 1:1 molar equivalents ratio.

**Example 14**

An ophthalmic device was made as described in Example 1 using EDC-NHS + chondroitin sulfate C (ChS) + collagens at pH 7-8 in PBS buffer, at 0° - 4°C raised to 21°C for 15 h, then 15 h at 37°C. EDC:NHS = 1:1 molar equivalents ratio.

All devices of Examples 3-14 gave robust, clear and flexible gels with all of the commercial collagens and reactant ratios indicated in Table 2, below.

Some hydrogels for onlay applications were characterized by DSC, optical clarity and refractive index, measurements, tensile properties (stiffness, maximum tensile strength, elongation at break, Table 2) and in vivo performance. From DSC measurements on gels after reaction for all examples, an increase in the denaturing temperature and a decrease in $\Delta H_{denature}$ were found, consistent with cross-linking of the collagen. Refractive indices of all formulations in Table 2 were in the 1.341 to 1.349 range.

**Example 15**

In vitro onlay performance (Table 2)

The methods disclosed by Li et al. PNAS 100:15346-15351 (2003) were used to evaluate how epithelial cells (human, immortalized corneal epi. cells, HCEC) grow to confluence over hydrogels (days to confluence), to evaluate how HCEC cells stratify over hydrogels, and to evaluate chick dorsal root ganglion nerve growth over and into hydrogels (latter reported as micron/day growth where data available).

Human corneas restore their epithelium in 3 - 5 days after complete removal.

In vitro test duration was usually about 6 -8 days, but the better formulations allowed re-epithelialisation to confluence within 3 - 5 days or less. For denser gels (>5% collagen), extensive nerve over growth (300 micron extension) was found in the in vitro tests for many formulations. Nerve in-growth slows rapidly as gel stiffness increases, but was seen by in-depth microscopy.

Table 2. Composition and performance of hydrogels[†]

| Example # | Collagen supplier, (Table 1) (initial concn. wt/vol %) | Collagen/XL Equiv. Ratio or (wt/wt) | Final collagen concentration in the gel (w/v %) | Maximum stress, g force* | Strain at break, mm* | Stiffness, g/mm* | In vitro Epi. cells, days to confluence | In vitro Nerve growth in 6 days** |
|---|---|---|---|---|---|---|---|---|
| 2 | B AFDP: (dissolved at 10%) | Col-$NH_2$:EDC=5:1 Col:YIGSR = 5: 0.0001 | 7.2 | | | | 3-5 | Over: fast. In:27μm/d |
| 3 | A, (10% bovine) | Col-$NH_2$:EDC = 5:1 | 7.3 | 8.0 | 2.6 | 4.0 | | |
| 3 | B, AFDP: (dissolved at 10%) | Col-$NH_2$ :EDC = 5:1 | 7.3 | 9.7 | 4.0 | 4.4 | 2-3 | Over: fast In: 40 μm/d |
| 3 | B, AFDP: (dissolved at 15%) | Col-$NH_2$ :EDC = 5:1 | 10.8 | 13.08 | 4.6 | 3.0 | 2-3 | |
| 3 | B, AFDP:(dissolved at 20%) **350 μm** thickness gel | Col-$NH_2$ :EDC = 10:1 | 14.3 | 11.95 | 4.3 | 3.0 | 2-3 | |
| 3 | B, (AFDP, dissolved at 32%) | Col-$NH_2$:EDC = 1:1 | 18.0 | 14 | | | 2-3 | Over: fast In: = 30 μm/d |
| 3 | A, (3.5% neutral bovine | Col-$NH_2$:EDC =1:1 | 2.7 | 3.1 | 2.0 | 1.7 | 3-5 | Over: fast |
| 5 | A, (3.5% neutral bovine) | Col-$NH_2$:EDC = 2:1 Col:ChS= (9:1) | 2.7 | 2.5 | 1.8 | 1.4 | 3 | Over: fast In: =41 μm/d |
| 5 | A, (3.5% neutral bovine) | Col-$NH_2$:EDC = 2:1 Col:ChS= (4:1) | 2.7 | 2.7 | 1.8 | 1.5 | 3 | Over : fast In: =73 μm/d |
| 5 | A, (3.5% neutral bovine) | Col-$NH_2$:EDC = 2:1 Col:ChS= (3:1) | 2.7 | 3.1 | 1.5 | 1.5 | 3 | Over In: =70 μm/d |

| Example # | Collagen supplier, (Initial concn. Wt/vol %) | Collagen/XL Equiv. Ratio or (wt/wt) | Final collagen concentration in the gel (w/v %) | Maximum stress, g force* | Strain at break(mm)* | Stiffness, g/mm* | In vitro Epi. growth, days to confluence | In vitro Nerve growth in 6 days** |
|---|---|---|---|---|---|---|---|---|
| 6 | A, (3.5% neutral bovine) | Col-NH$_2$ :EDC =1:1<br>Col:CMC = (1:0.5) | | 3.6 | 1.6 | 2.0 | | |
| 6 | B, (AFDP, dissolved at 32%) | Col-NH$_2$:EDC = 1:1.3<br>Col:CMC =(15:1) | 14.5 | 6.0 | | | 3 | |
| 7 | A, (3.5% neutral bovine) | Col-NH$_2$:EDC = 1:1<br>Col:CMC =(2:1) + soluble chitosan | | 2.9 | 1.6 | 1.9 | | |
| 8 | A, (3.5% neutral bovine) | Col-NH$_2$:EDC = 2:1<br>Col:HA= (9:1) | 2.2 | 2.5 | 2.2 | 1.08 | 3-5 | |
| 8 | A, (5% neutral bovine) | Col-NH$_2$:EDC = 2:1<br>Col:HA= (4:1) | 2.2 | 2.4 | 2.2 | 2.07 | 3-5 | |
| 8 | A, (10% neutral bovine) | Col-NH$_2$:EDC = 2:1<br>Col:HA= (3:1) | 2.2 | 2.0 | 1.8 | 1.13 | 3-5 | |
| 9 | A, (3.5% neutral bovine) | Col-NH$_2$:EDC = 0.5:1.0<br>Col:HA:ChS=9: 1:1 | 2.3 | 3.0 | 1.7 | 1.7 | 3-5 | Over and in growth |
| 10 | A, (3.5% neutral bovine)<br>**350 μm** thickness gel | Col-NH2: HA-CHO = 1:1 | 3.2 | 1.0 | | 0.7 | | |
| 11 | A, (3.5% neutral bovine | Col-NH$_2$:EDC = 2:1<br>Col:Alg = 4:1 | 2.7 | 3.0 | 2.0 | 1.6 | 3-5 | Over: fast<br><br>In: = 41 μm/d |

EP 1 776 148 B1

(continued)

| Example # | Collagen supplier, (Initial concn. Wt/vol %) | Collagen/XL Equiv. Ratio or (wt/wt) | Final collagen concentration in the gel (w/v %) | Maximum stress, g force* | Strain at break(mm)* | Stiffness, g/mm* | In vitro Epi. growth, days to confluence | In vitro Nerve growth in 6 days** |
|---|---|---|---|---|---|---|---|---|
| 11 | A, (3.5% neutral bovine) | Col-NH$_2$:EDC = 2:1<br>Col:Alg = 2:1 | 2.7 | 3.4 | 2.5 | 1.5 | 3-5 | Over: fast<br><br>In: = 13 μm/d |
| 12 | A, (3.5% neutral bovine) | Col:Glut= (130:1)?? + soluble chitosan | | 3.1 | 2.1 | 1.5 | | |
| Example # | Collagen supplier, (Initial concn. Wt/vol %) | Collagen/XL Equiv. Ratio or (wt/wt) | Final collagen concentration in the gel (w/v %) | Maximum stress, g force* | Strain at break (mm)* | Stiffness, g/mm* | In vitro Epi. growth, days to confluence | In vitro Nerve growth in 6 days** |
| 13 | B, (11% AFDP), 900 μm thickness gel | Col-NH$_2$:EDC = 0.33:1.0<br>Col:chitosan =(15:1) | 5.8 | 8.32 | 3.29 | 2.57 | 4 | |
| 13 | B, (11% AFDP), 500 μm thickness gel | Col-NH2:EDC = 0.66:1.0<br>Col:chitosan =(15:1) | 5.8 | 4.25 | 4.02 | 1.32 | | |
| 13 | B, (11% AFDP), 900 μm thickness gel | Col-NH$_2$:EDC = 0.66:1.0<br>Col:chitosan =(15:1) | 5.8 | 8.46 | 5.23 | 2.17 | | |

†Abbreviations: Col collagen; Glut glutaraldehyde; HA hyaluronic acid; ChS chondroitin sulfate C; Col-NH$_2$ free amine content of collagen; AFDP acid, freeze dried porcine; epi. epithelial; ND not determined.
*500 μm thick, 12 mm diameter implants unless otherwise indicated. Stress, strain and stiffness data from the suture pull out method as disclosed by Li et al. PNAS 100: 15346-15351 (2003).
**Over: neurites from DRG overgrew hydrogel. In: neurites grew into the hydrogel to the indicated length in 6 days.

EP 1 776 148 B1

**EXAMPLE 16**

In vivo onlay performance

Onlays were prepared as described in Example 1. A first set of onlays were prepared from 10% (w/v) porcine collagen with EDC/NHS. A second set of onlays were prepared from 3.5% (w/v) bovine collagen with chrondoitin sulphate (CSC) and EDC/NHS. The onlays had a diameter of about 6 mm, a center thickness of about 70 $\mu$m, and 30 $\mu$m sloped edges.

To implant the onlays, the epithelium of a pig was treated with 45% ethanol for 30-45 seconds. A butterfly incision was made and a pocket was formed in the epithelium. The onlays were stained with blue non-cytotoxic dye (Gel-Code™) for visualization. The pre-stained onlays were inserted into the pocket. A protective contact lens was sutured over the eye.

Visual inspection was performed to evaluate inflammation, redness, and/or vascular invasion of the cornea. Slit lamp examinations were used to assess corneal clarity. A tonopen was used to measure intraocular pressure. A Cochet-Bonnet aesthesiometer was used to determine touch sensitivity of the cornea. The touch sensitivity may be useful to evaluate the presence of functional nerves, which was corroborated with in vivo confocal imaging and immunohistochemistry of the harvested corneas with implants. Corneal topography was examined with a PAR Corneal Topography System (CTS) immediately before implantation and three weeks after surgery.

Corneal topography was performed by aligning an eye of an anesthetized pig with the CTS. A dilute solution of fluoroscein and artificial tears was applied to the eye to coat the corneal surface and enable visualization of a target grid. The instrument focal plane was adjusted to bring the target grid into focus on the anterior corneal surface. A digital image of the grid was captured. The digital image was analyzed to provide a measure of the shape of the anterior corneal surface. Comparing the digital image before and after the implantation of the onlay can be used to evaluate changes in the shape of the cornea due to the placement of the onlay.

In vivo confocal microscopy permits images of different depths of the cornea in live pigs to be captured, and thus allows the response of the eye to the ophthalmic device to be monitored. For example, the confocal microscopy can be used to monitor the presence of nerves in the device. In vivo confocal microscopy was performed by examining anesthetized pigs with a Nidek Confoscan 3 in vivo confocal microscope before implantation of the ophthalmic device and 3 weeks after the surgery. Artificial tears were placed on the eye to be examined. Two drops of a local anesthetic were applied to the eye to reduce eyeball movement. The confocal lens (gel immersion) was brought into contact with the cornea with a layer of gel on the front surface of the lens for refractive index matching. The instrument focal plane was adjusted to bring the corneal endothelium into focus, and then images of the cornea were taken as the focal plane of the lens was scanned through a depth equal to the thickness of the cornea.

Immunohistochemistry was used in addition to histopathological examination of hematoxylin and eosin (H&E) stained tissue sections to determine if corneal epithelial recovery over the onlay and early indication of adhesion and interaction with the underlying onlays. Immunohistochemistry was also used to establish the presence or absence of nerves and any infiltration of immune and inflammatory cells. Anti-neurofilament staining was performed on half corneas, with and without implanted onlays after permeabilization with detergent, using conventional techniques. Immunofluorescence was used to visualize bound antibody.

Corneas that received a corneal onlay, as discussed above, healed well and remained optically clear with minimal or no redness or inflammation. There were no signs of vascular infiltration. Normal intraocular pressures were observed. The post-operative corneas exhibited touch sensitivity. Topograph measurements indicated that the implanted onlays were able to effect changes in corneal topography. The onlays resulted in a change of about 50 $\mu$m in thickness in central corneal elevation. The epithelium adhered well to the onlays. In vivo corneal microscopy revealed good general corneal structure with subepithelial and stromal nerves, and cells from the epithelium through to the endothelium. H&E stained cryo-sectons showed integration of the onlays into the host cornea. Immunohistochemistry demonstrated little, if any, change in the adhesivity of the cells in onlay-implanted corneas compared to untreated corneas using a stain for E-cadherin. Staining for Keratin 3 and E-cadherin was comparable to controls. Collagen type VII staining for anchoring fibers with the basement membrane complex showed less distinct staining than controls. Staining for $\alpha$6 integrin showed localization in the basal epithelial cells in both operated and untreated controls. Anti-neurofilament 200 antibody staining showed the presence of nerves in the implantation site in corneas with onlays. Anti CD 45 antibody staining revealed no inflammatory or immune reaction.

**EXAMPLE 17**

Ablation of corneal onlays

Collagen/EDC and collagen/chitosan onlays were ablated using a VISX Star S4 excimer laser (Table 3). Surface topography measurements of the onlays were obtained before and after treatment with a PAR Corneal Topography System (CTS). For the treatment, the onlays were removed from the storage solution and laid on a spherical surface made of PMMA.

Phototherapeutic keratectomy (PTK) surgery delivers a uniform number of laser pulses (or energy) to an entire ablation zone. Photorefractive keratectomy (PRK) surgery varies pulse density over the ablation zone to achieve a desired change in curvature. AZD refers to ablation zone diameter. Depth is the predicted depth of the treatment on a human cornea as reported by the laser manufacturer.

TABLE 3: Ablation Parameters.

| Surgery | Type | AZD (mm) | Sphere (D) | Cyl (D) | Depth (μm) |
|---------|------|----------|------------|---------|------------|
| 1 | PTK | 5 | ---- | ---- | 10 |
| 2 | PTK | 5 | ---- | ---- | 20 |
|  |  |  |  | - |  |
| 3 | PRK | 6 | 2 | 0 | 26 |
|  |  |  | +2 |  |  |
| 4 | PRK | 6 |  | 0 | 19 |
|  |  |  |  | - |  |
| 5 | PRK | 6 | 4 | 0 | 51 |
|  |  |  | +4 |  |  |
| 6 | PRK | 6 |  | 0 | 38 |
|  |  |  | - | +2 |  |
| 7 | PRK | 6 | 4 |  | 30 |

Difference maps were generated from the pre- and post-operation topographies of the collagen/EDC onlays to display the effects of the ablation.

The PTK ablations were expected to produce a fairly constant central blue region ~5 mm in diameter (for the difference map). A small gradient in the amount of tissue removed was expected since the onlay is a curved surface. Myopic sphere PRK ablations were expected to remove the maximum tissue depth centrally. The depth of tissue removed was expected to gradually decrease to zero at the edge of the ablation. Hyperopic sphere corrections were expected to leave the central 1 mm diameter untouched and remove tissue maximally at the edge of the treatment zone, a transition zone was also expected to be created peripherally out to 9 mm from the center. Difference maps after a hyperopic correction were expected to display a ring of blue around a central green zone. The difference map from the myopic astigmatic correction was expected to appear similar to the map from myopic spherical correction except its blue pattern was expected to be elliptical.

The difference maps from the ablated onlays demonstrated the aforementioned expected tissue removal patterns in all the difference maps. The maximum depth of tissue removed appeared to be greater than that predicted for the human cornea. For example, the rate at which the corneal onlay material was removed was between about 1.7 and about 2 times the rate of corneal removal. The difference between the ablation rate of the onlay material and a cornea was not uniform across the samples. The rate differences may be due to, among other things, the depth of treatment measurement, material densities and surface roughness, and water content of the material.

The collagen/chitosan onlays were observed to ablate at a faster rate then the collagen/EDC onlays. The difference could be due to hydration issues. For example, the post-operative collagen/chitosan onlays may have had a lower water content than the collagen/EDC onlays.

The ophthalmic device may also comprise a strength increasing component, such as urethane.

**EXAMPLE 18**

Human recombinant collagen ophthalmic devices

Cross-linked collagen hydrogels were prepared by mixing 0.3 ml of 13.7 wt% human recombinant type I collagen obtained from FibroGen (San Francisco, CA) and 0.3 ml of 0.625 M morpholinoethanesulfonic acid (MES) using a syringe based system as described herein. The mixing occurred at a reduced temperature by performing the mixing in an ice-water bath.

After a homogenous solution was obtained, 57 $\mu$l of EDC/NHS was injected into the mixture in a molar equivalent ratio to collagen free amine (coll-NH$_2$) groups of 3:3:1. To adjust the pH of the solution to about 5, NaOH (2N) was added to the mixture.

The mixture was cast into glass or plastic molds and left at room temperature with 100% humidity for 16 hours. The molds were subsequently transferred into an incubator for post-curing at 37°C for 5 hours.

Other hydrogels with human recombinant collagen type I with ratios of EDC/NHS to collagen coll-$NH_2$ groups of 1:1:1 and 6:6:1 were also prepared using this method.

Refractive index (RI) was determined on a VEE GEE refractometer. Optical transmission was measured at wavelengths of white light, 450 nm, 500 nm, 550 nm, 600 nm, and 650 nm. Direct tensile property measurements, such as stress, break strain, and moduli, were determined on an Instron electromechanical tester (Model 3340). The size of the samples were 5 mm x 5 mm x 0.5 mm. The water content of the hydrogels were calculated according the following equation:

$$(W-W_0)/W\%$$

where $W_0$ and W denote weights of dried and swollen samples, respectively.

A human recombinant collagen hydrogel with a ratio of EDC/NHS/Coll-$_{NH2}$ of 1/1/1 (molar equivalents) (designated F1) had a refractive index of $1.3457\pm0.0013$. A human recombinant collagen hydrogel with a ratio of EDC/NHS/Coll-$_{NH2}$ of 3/3/1 (molar equivalents) (designated F3) had a refractive index of $1.3451\pm0.0002$. A human recombinant collagen hydrogel with a ratio of EDC/NHS/Coll-$_{NH2}$ of 6/6/1 (molar equivalents) (designated F6) had a refractive index of $1.3465\pm0.0001$.

Table 4 summarizes the optical transmission of the different hydrogels.

TABLE 4. Optical Transmission

| Wavelength(nm) | White | 450 | 500 | 550 | 600 | 650 |
|---|---|---|---|---|---|---|
| Average Transmission (%) | | | | | | |
| F1 | 86.7±0.9 | 69.7±1.2 | 76.0±1.3 | 79.2±1.4 | 82.4±1.3 | 84.9±1.4 |
| F3 | 90.7±2.5 | 85.8±3.5 | 86.4±2.9 | 86.7±2.6 | 88.0±2.4 | 89.6±2.6 |
| F6 | 75.5±1.5 | 48.7±0.4 | 57.7±1.1 | 62.7±1.1 | 67.6±1.4 | 71.6±1.7 |

The hydrogel material designated F3 appeared to show the most acceptable optical properties. Visually, or macroscopically, F3 appeared to have the greatest transparency compared to the other human recombinant hydrogels.

Table 5 provides mechanical properties of the present human recombinant hydrogels.

TABLE 5. Mechanical Properties

| Samples | F1 | F3 | F6 |
|---|---|---|---|
| Average Maximum Stress(KPa) | 62.6± 9.9 | 117.2±36.9 | 149.9±57.7 |
| Average Break Stress(KPa) | 67.1± 21.0 | 110.5±49.7 | 99.5±60.8 |
| Average Break Strain(%) | 62.60± 6.82 | 50.20±7.55 | 23.51±9.03 |
| Average Modulus(MPa) | 0.281± 0.032 | 0.525±0.124 | 1.949±0.939 |

The hydrogel F3 appears to have a relatively low modulus, but acceptable other mechanical properties.

Table 6 provides water content values for the hydrogel materials.

TABLE 6. Equilibrated Water Contents

| Samples | F1 | F3 | F6 |
|---|---|---|---|
| Water content (%) | 92.82±0.68 | 92.63±0.61 | 91.40±0.38 |

It is clear that the hydrogels are highly hydrated.

In the present example, a pH indicator was added to the MES buffer to help monitor pH changes. The particular indicator used in this example is Alizarin Red S (Sigma Aldrich).

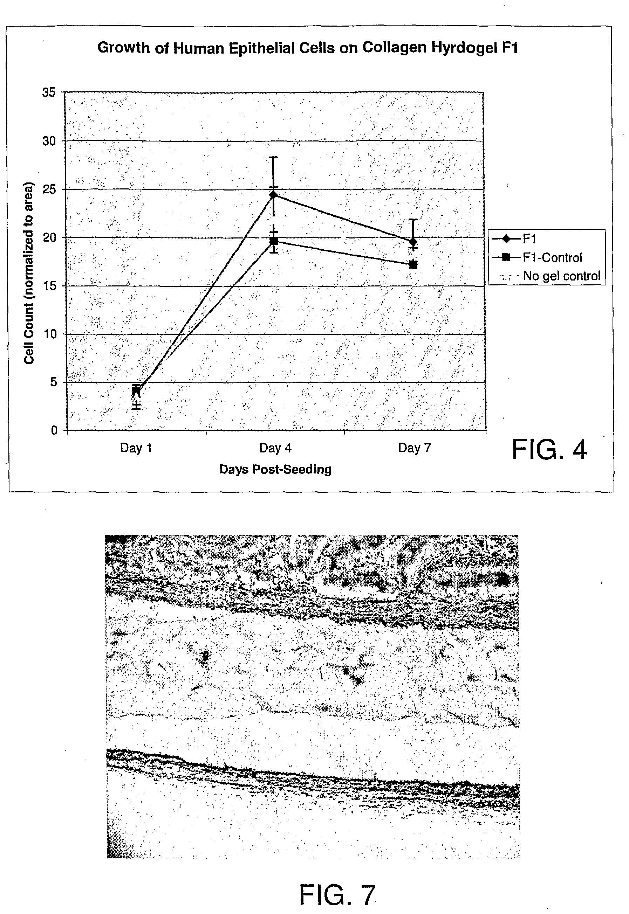

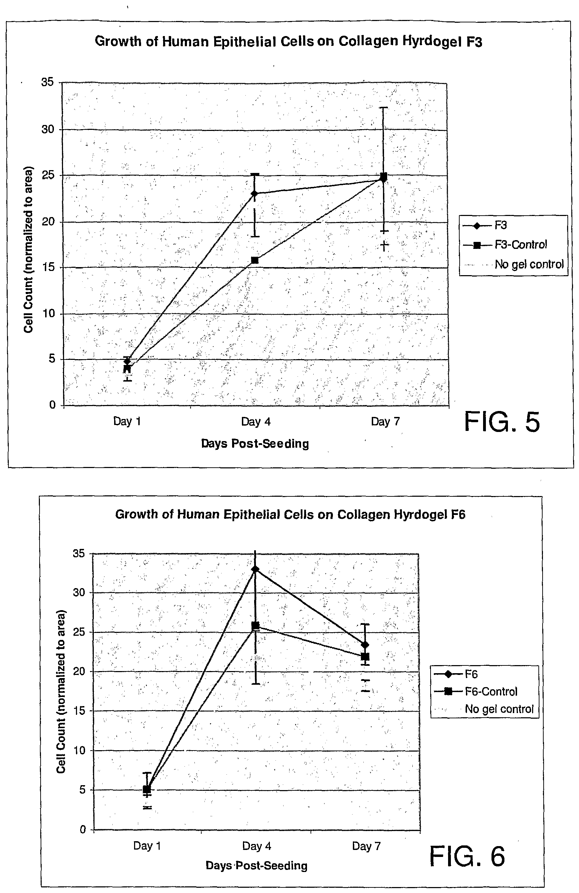

FIG. 4 is a graph of human corneal epithelial cell growth on the human recombinant collagen sample F1 over a 7 day period. FIG. 5 is a graph of human corneal epithelial cell growth on the human recombinant collagen sample F3 over a 7 day period. FIG. 6 is a graph of human corneal epithelial cell growth on the human recombinant collagen sample F6 over a 7 day period.

The cell growth observed on the recombinant hydrogel materials was greater than that observed for the control experiments.

FIG. 7 is a photograph demonstrating that the hydrogel material F3 persisted in vivo for at least 30 days.

**EXAMPLE 19**

Collagen-poly(NIPAAm-co-AAC) compositions

A composition was prepared using the EDC/NHS cross-linking methods described herein (Example 4). The starting collagen concentrations was 15%. The final collagen concentration was 11%. The final poly(NIPAAm-co-Aac) concentration was 3%. The total solid concentration in the gel was 14%.
This material had a refractive index of 1.3542, a tensile strength of 11g-force, an elongation of 3.3 mm, and a modulus of 3.8 g-force/mm from the suture pull out method as disclosed by Li et al. PNAS 100:15346-15351 (2003).

The denature temperature increased from 40°C before crosslinking to 50°C after crosslinking. The material had a higher optical transmission and lower back scatter than human cornea or rabbit cornea. For example, with white light, the percent transmission was about 102% for the hydrogel material, and about 93% for the human cornea, and 78% for the rabbit cornea. The hydrogel had percent transmissions of about 90%, 96%, 100%, 101%, and 103% for wavelengths of light of 450 nm, 500 nm, 550 nm, 600 nm, and 650 nm, respectively. The human cornea and the rabbit cornea exhibited percent transmissions less than 100% at all tested wavelengths, and exhibited percent transmission that were consistently lower than the hydrogel material at each wavelength.

The hydrogel material demonstrated corneal epithelial cell confluence at seven days after seeding.

**EXAMPLE 20**

Collagen/chondroitin sulfate compositions

Biosynthetic matrices with high optical clarity and tensile strength were developed using collagen I, with chondroitin sulphate (CSC) as a proteoglycan equivalent. Hydrogels with up to 30% (wt/wt) CSC to collagen dry weight were prepared under controlled conditions, without coagulation or collagen fibrillogenesis which can cause loss of optical clarity. The hydrogels were characterized physically and biochemically. The in vitro test showed that human corneal epithelial cells (HCECs) grew well on gels' surfaces and stratified successfully. The matrix supported good nerve in growth. Similar results were also obtained in vivo.

Compositions were prepared similar to Example 14 described herein. CSC was covalently bound to collagen using EDC and NHS chemistry.

Collagen (3.5 w/v%) and CSC gels with different CSC to collagen dry weight ratios and different EDC to collagen-NH2 mole equivalent ratios were prepared by using EDC/NHS (1:1 mole equivalent) cross-linking techniques, as discussed herein. All gels were visually transparent. The compositions had higher light transmission and low light scatter compared to human cornea (about 87% transmission and 3% back scatter), as shown in Table 7.

TABLE 7-Transmission and Light Scatter

| CSC to collagen weight ratio (%) | **0** | **5** | **10** | **20** | **30** |
|---|---|---|---|---|---|
| Transmission (%) | 89.9 | 95.5 | 93.0 | 90.5 | 97.3 |
| Back Scatter (%) | 0.30 | 0.19 | 0.19 | 0.17 | 0.19 |
| EDC to NH$_2$ ratio | **0.25** | **0.5** | **1.0** | **2.0** | |
| Transmission (%) | 96.7 | 100 | 99.9 | 88.2 | |
| Back Scatter (%) | 0.24 | 0.28 | 0.16 | 0.20 | |

Refractive indices were between 1.34-1.35, which is close to refractive index of the human cornea (1.376).

The swelling ratio of the gels made by using different EDC to collagen-NH$_2$ mole ratios were measured and calculated by the following equation:

$$\text{Swelling ratio} = (W_w - W_d)/W_d$$

where $W_w$ is the weight of the hydrated gel and $W_d$ is the weight of dry gel.

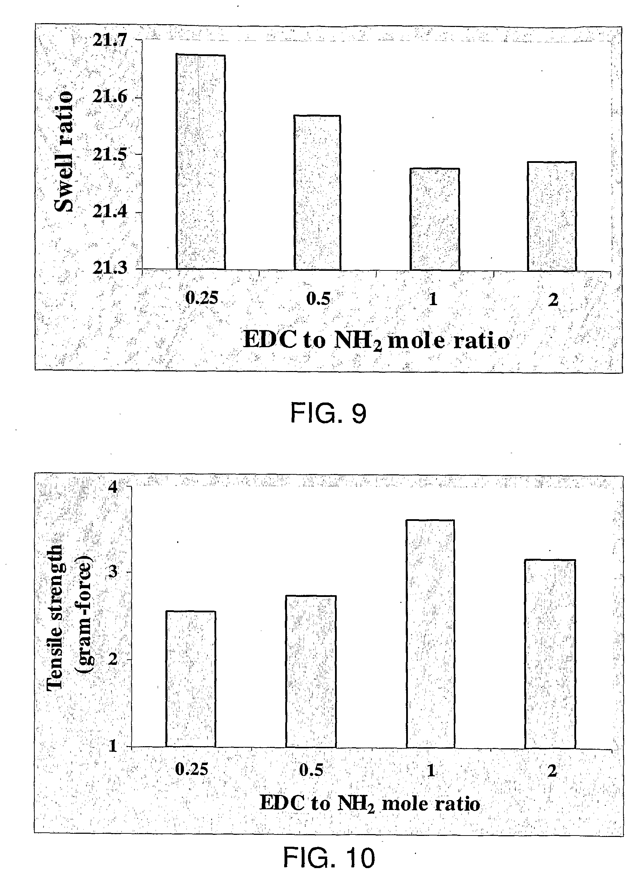

Collagen-CSC crosslinking using EDC/NHS results in the formation of crosslinks between carboxylic acid and amine groups. The results (FIG. 9) suggested that increasing EDC to collagen-$NH_2$ ratios decreased the swelling ratio of the collagen-CSC gel because it introduced more condensed networks.

Mechanical properties measurement of the implants were performed by the suture pull out method as disclosed by Li et al. PNAS 100:15346-15351 (2003). The implants were fully hydrated in PBS and drawn at a speed of 10mm/min. The tensile strength was monitored at rupture of the implant (500$\mu$m in thickness and 12mm in diameter). Tensile strength of the gels was enhanced by increasing the EDC to $NH_2$ mole ratio (FIG. 10). However, the material became brittle when the amount of EDC was increased substantially, thus the tensile strength decreased a little when the EDC to collagen-$NH_2$ mole ratio was 2 or higher.

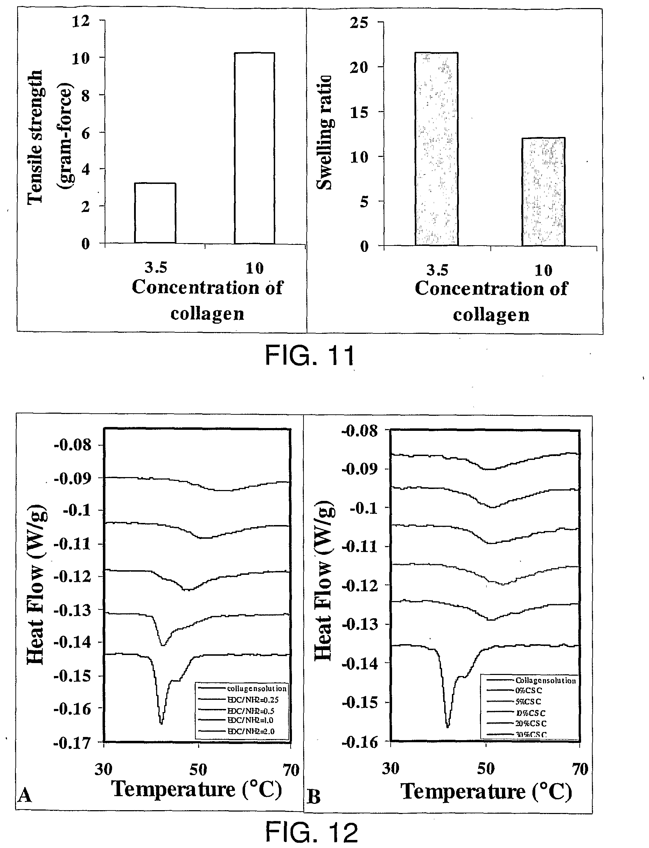

The tensile strength of the gel can also be enhanced by increased concentration of collagen, as shown in FIG. 11 (cf. use 10% collagen instead of 3.5%). The tensile strength was increased from 2.65 to 10.02 gram-force and the swelling ratio decreased from 21.5 to 12.1.

The efficiency of the crosslinking was evaluated by differential scanning calorimetry (DSC). Heating collagen or crosslinked collagen hydrogels will induce a structural transition of the native triple helical structure at a temperature depending on the nature and the degree of the crosslinking. Collagen solution and crosslinked fully hydrated, collagen hydrogels were characterized in a hermetically sealed pan and temperature of the samples was raised at a constant rate of 2°C/min. The temperature at the maximal peak was recorded as the denature temperature. As the EDC to $NH_2$ ratio was increased, the denature temperature increased from 42.4°C to 56.6°C (FIG.12A), which suggested that introduction of covalent crosslinks increased the stability of the triple helix, and thus increased denature temperature. The denature temperature of the collagen-CSC gel was higher than the collagen only gel (FIG.12B). However, changing the CSC to collagen mole ratio in collagen-CSC gels did not affect the denature temperature.

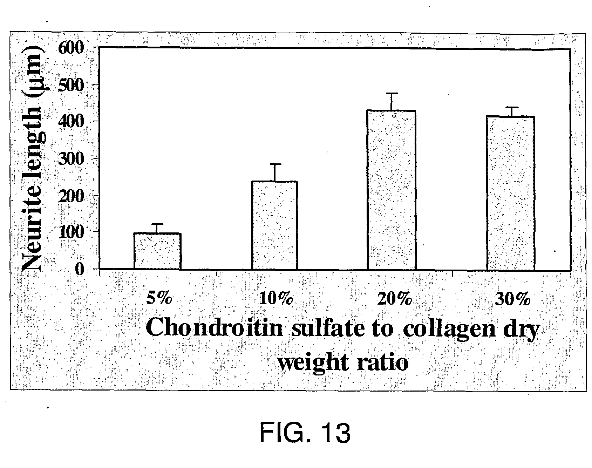

In vitro growth of human corneal epithelial cells from an established cell line was observed. Nerve growth was performed in vitro, using dorsal root ganglia implanted into the gel. Neurites were grown for 7 days, the gels were stained for neurofilament and neurite extension was measured. Neurites grew well in all collagen-CSC gels (FIG. 13).

Increasing the concentration of CSC from 5% to 20% greatly enhanced the length of neurite extension within the gels. No additional benefit was apparent in gels containing 30% CSC (Fig. 13). Excellent epithelial coverage and implant integration were observed.

**Example 21**

Type III Collagen Compositions

**Materials.** Human recombinant type III collagen (5.1% w/w FibroGen Inc), 0.625 M morpholinoethanesulfonic acid [MES, containing Aalizarin Red S pH indicator(6.5 mg/100ml water)], 1-ethyl-3-(3-dimethyl aminopropyl) carbodiimide HCl (EDC), N-hydroxy-succinimide(NHS).

Hydrogels were made from 18.3% (w/w) type III collagen solution. 0.3 ml of 18.2 wt% human recombinant type III collagen (concentrated from 5.1% w/w human recombinant type III collagen FibroGen Inc) and 0.3 ml of MES(0.625 M) were mixed in two bubble free syringes connected with a plastic Tee in ice-water bath. After a homogenous solution was formed, 33.5mg of EDC and 20.1 mg of NHS were dissolved in 0.125 ml of MES, of which 57 $\mu$l was taken and injected into the above syringes in molar ratio of EDC:NHS:collagen-$NH_2$ of 3:3:1. No NaOH solution was added since the mixture appeared pink, indicating pH around 5. The mixture was mixed thoroughly and cast into glass moulds (thickness 434$\mu$m) and left at room temperature, at 100% humidity, for 16 h. Then the moulds were transferred into an incubator for post-curing at 37 °C for 5 h. The resulting flat hydrogels were removed and soaked in 10 mM PBS with fresh buffer being replaced at 8 h intervals. The hydrogels obtained were immersed in 10 mM PBS containing 1% chloroform and stored in 4 °C refrigerator.

Addtional type III collagen hydrogels with ratios of EDC:NHS:collagen-NH2 of 2:2:1 and 1:1:1 were also prepared using the above-described method. All the gels obtained were transparent.

Hydrogels were also made from 5.1%(w/w) type III collagen solution. 0.3 ml of 5.1 wt% human recombinant type III collagen and 50$\mu$l of MES (0.625 M) were mixed in two bubble free syringes connected with a plastic Tee in ice-water bath. After a homogenous solution was formed, 9.3mg of EDC and 5.6 mg of NHS were dissolved in 0.125 ml of MES, of which 57 $\mu$l was taken and injected into the above syringes in molar ratio of EDC:NHS: collagen-$NH_2$ of 3:3:1. No NaOH solution was added since the mixture appeared pink, indicating pH around 5. The mixture was mixed thoroughly and cast into glass moulds (thickness 434$\mu$m) and left at room temperature with 100% humidity for 16 h. Then the moulds were transferred into an incubator for post-curing at 37 °C for 5 h. The resulting flat hydrogels were taken out and soaked in 10 mM PBS with fresh buffer being replaced at 8 h intervals. Lastly the hydrogels obtained were immersed in 10 mM PBS containing 1% chloroform and stored in 4 °C refrigerator. The resultant gel was optically clear.

The final collagen contents for 18.3% w/w collagen starting concentration was 8.36 %(w/v)(calculated based on the dilution factors after added every components) or approximately 10% (w/v) (measured).

The final collagen contents for 5.1% w/w collagen starting concentration was 3.76 %(w/v)(calculated based on the dilution factors after added every components) or approximately 4% (w/v) (measured).

**Claims**

1. An optically clear biosynthetic, implantable corneal device, comprising cross-linked collagen, wherein the cross-linked collagen is obtainable by a process of cross-linking collagen polymers using 1-ethyl-3-(3-dimethylaminopropyl) carbodiimide (EDC) and N-hydroxysuccinimide (NHS), and wherein the composition comprises an amount of collagen between 5% and 50% by weight or volume

2. The device of claim 1, wherein collagen is the sole polymer.

3. The device of claim 1, wherein collagen is the sole water-swellable polymer.

4. The device of claim 1, wherein collagen is the sole lens-forming polymer.

5. The device of any one of the preceding claims, wherein the amount of collagen is

   (a) at least 6% by weight or volume;
   (b) at least 10% by weight or volume;
   (c) between 10% and 30% by weight or volume; or
   (d) between 10% and 24% by weight or volume.

6. The device of any one of the preceding claims, wherein the cross-linked collagen comprises:

   (a) one type of collagen; or
   (b) two or more types of collagen.

7. The device of any one of the preceding claims, which further comprises a cell growth enhancer agent.

8. The device of claim 7, wherein the cell growth enhancer agent is a peptide or is selected from neurotrophic factors, nerve growth factors, and epidermal growth factors.

9. The device of claim 8, wherein the peptide has an amino acid sequence of RGD, YIGSR, or IKVAV.

10. The device of claim 8, wherein the cell growth enhancer agent is distributed substantially throughout the device.

11. The device of claim 1, wherein the collagen:

    (a) is the sole water-swellable polymer of the device; and/or
    (b) was cross-linked at an acidic pH.

12. The device of claim 11, wherein the acidic pH is between 5.0 and 5.5.

13. The device of any one of the preceding claims, wherein the cross-linked collagen comprises:

    (a) atelocollagen, type I collagen, type III collagen, or a combination thereof;
    (b) recombinant collagen; or
    (c) collagen isolated from an animal.

14. The device of any one of the preceding claims, wherein the cross-linked collagen is produced by mixing together the collagen polymers and the EDC/NHS cross-linker at an acidic PH while preventing surges in pH.

15. The device of any one of the preceding claims, wherein the mixing of polymers and crosslinker agent is at high shear.

16. The device of claim 1, which further comprises chondroitin sulfate, N,O-carboxymethylchitosan, hyaluronic acid, hyaluronic acid aldehyde or alginate.

17. A method of making a composition according to any one of claims 1 to 16, comprising:

combining collagen polymers using 1-ethyl-3-(3-dimethylaminopropyl)carbodiimide and N-hydroxysuccinimide cross-linker agents at an acidic pH; and
curing the resultant combination to form the composition comprising cross-linked collagen.

18. The method of claim 17, wherein:

(a) the combining step comprises mixing the collagen polymers and the cross-linker agent in a system configured to produce high shear forces on the resultant combination; and
(b) the combining occurs at a temperature between 0°C and 5°C.

19. The method of claim 17 or 18, further comprising adding a cell growth enhancer agent to the combination.

20. A device according to any one of claims 1 to 16 for use in the treatment of an ophthalmic disease, disorder or injury in a subject in need thereof.

21. A device according to claim 20 wherein an eye of the subject having said ophthalmic disease, disorder or injury is contacted with said ophthalmic device.

22. A device according to any one of claims 1 to 16 for use in facilitating nerve growth on and/or into the device.

**Patentansprüche**

1. Optisch klare biosynthetische, implantierbare korneale Vorrichtung, umfassend quervernetztes Kollagen, wobei das quervernetzte Kollagen durch einen Prozess erhalten werden kann, bei dem Kollagenpolymere quervernetzt werden unter Verwendung von 1-Ethyl-3-(3-dimethylaminopropyl)carbodiimid (EDC) und N-Hydroxysuccinimid (NHS) und wobei die Zusammensetzung eine Kollagenmenge zwischen 5 und 50 Gew.- oder Vol.-% umfasst.

2. Vorrichtung nach Anspruch 1, wobei Kollagen das einzige Polymer ist.

3. Vorrichtung nach Anspruch 1, wobei Kollagen das einzige wasserquellbare Polymer ist.

4. Vorrichtung nach Anspruch 1, wobei Kollagen das einzige linsenbildende Polymer ist.

5. Vorrichtung nach einem der vorangehenden Ansprüche, wobei die Menge von Kollagen Folgendes ist:

(a) mindestens 6 Gew.- oder Vol.-%;
(b) mindestens 10 Gew.- oder Vol.-%;
(c) zwischen 10 und 30 Gew.- oder Vol.-%; oder
(d) zwischen 10 und 24 Gew.- oder Vol.-%.

6. Vorrichtung nach einem der vorangehenden Ansprüche, wobei das quervernetzte Kollagen Folgendes umfasst:

(a) einen Kollagentyp; oder
(b) zwei oder mehr Kollagentypen.

7. Vorrichtung nach einem der vorangehenden Ansprüche, die ferner ein Zellwachstumsverstärkermittel umfasst.

8. Vorrichtung nach Anspruch 7, wobei das Zellwachstumsverstärkermittel ein Peptid ist oder aus neurotrophen Faktoren, Nervenwachstumsfaktoren und Epidermalwachstumsfaktoren ausgewählt ist.

9. Vorrichtung nach Anspruch 8, wobei das Peptid eine RGD-, YIGSR- oder IKVAV-Aminosäuresequenz hat.

10. Vorrichtung nach Anspruch 8, wobei das Zellwachstumsverstärkermittel im Wesentlichen über die Vorrichtung hinweg verteilt ist.

11. Vorrichtung nach Anspruch 1, wobei das Kollagen:

(a) das einzige wasserschwellbare Polymer der Vorrichtung ist; und/oder
(b) bei einem sauren pH-Wert quervernetzt war.

12. Vorrichtung nach Anspruch 11, wobei der saure pH-Wert zwischen 5,0 und 5,5 liegt.

13. Vorrichtung nach einem der vorangehenden Ansprüche, wobei das quervernetzte Kollagen Folgendes umfasst:

(a) Atelokollagen, Typ-I-Kollagen, Typ-III-Kollagen oder eine Kombination davon;
(b) rekombinantes Kollagen; oder
(c) Kollagen, das aus einem Tier isoliert wurde.

14. Vorrichtung nach einem der vorangehenden Ansprüche, wobei das quervernetzte Kollagen durch Zusammenmischen der Kollagenpolymere und des EDC/NHS-Quervernetzungsmittels bei einem sauren pH-Wert produziert wird, während pH-Wert-Anstiege verhindert werden.

15. Vorrichtung nach einem der vorangehenden Ansprüche, wobei das Mischen der Polymere und des Quervernetzungsmittels bei hoher Scherung erfolgt.

16. Vorrichtung nach Anspruch 1, die ferner Chondroitinsulfat, N,O-Carboxymethylchitosan, Hyaluronsäure, Hyaluronsäurealdehyd oder Alginat umfasst.

17. Verfahren zum Herstellen einer Zusammensetzung nach einem der Ansprüche 1 bis 16, umfassend:

Kombinieren von Kollagenpolymeren unter Verwendung von 1-Ethyl-3-(3-dimethylaminopropyl)carbodiimid und N-Hydroxysuccinimid-Quervernetzungsmitteln bei einem sauren pH-Wert; und
Aushärten der resultierenden Kombination, um die Zusammensetzung zu bilden, die quervernetztes Kollagen umfasst.

18. Verfahren nach Anspruch 17, wobei:

(a) der Kombinationsschritt ein Mischen der Kollagenpolymere und des Quervernetzungsmittels in einem System umfasst, das konfiguriert ist, um hohe Scherungskräfte auf die resultierende Kombination zu produzieren; und
(b) das Kombinieren bei einer Temperatur zwischen 0 °C und 5 °C stattfindet.

19. Verfahren nach Anspruch 17 oder 18, das ferner ein Hinzufügen eines Zellwachstumsverstärkermittels zu der Kombination umfasst.

20. Vorrichtung nach einem der Ansprüche 1 bis 16 zur Verwendung bei der Behandlung einer ophthalmischen Erkrankung, Krankheit oder Verletzung bei einem Subjekt, das dessen bedarf.

21. Vorrichtung nach Anspruch 20, wobei ein Auge des Subjekts mit der ophthalmischen Erkrankung, Krankheit oder Verletzung mit der ophthalmischen Vorrichtung in Kontakt gebracht wird.

22. Vorrichtung nach einem der Ansprüche 1 bis 16 zur Verwendung beim Vereinfachen von Nervenwachstum auf der und/oder in die Vorrichtung.

**Revendications**

1. Dispositif cornéen implantable biosynthétique optiquement transparent, comprenant le collagène réticulé, le collagène réticulé pouvant être obtenu par un processus de réticulation de polymères de collagène à l'aide de 1-éthyl-3-(3-diméthylaminopropyl) carbodiimide (EDC) et de N-hydroxysuccinimide (NHS), et la composition comprenant une quantité de collagène située entre 5 % et 50 % en poids ou volume.

**2.** Dispositif selon la revendication 1, dans lequel le collagène est le seul polymère.

**3.** Dispositif selon la revendication 1, dans lequel le collagène est le seul polymère gonflable à l'eau.

**4.** Dispositif selon la revendication 1, dans lequel le collagène est le seul polymère de formation de lentille.

**5.** Dispositif selon l'une quelconque des revendications précédentes, dans lequel la quantité de collagène est

(a) d'au moins 6 % en poids ou volume ;
(b) d'au moins 10 % en poids ou volume ;
(c) entre 10 % et 30 % en poids ou volume ; ou
(d) entre 10 % et 24 % en poids ou volume.

**6.** Dispositif selon l'une quelconque des revendications précédentes, dans lequel le collagène réticulé comprend :

(a) un type de collagène ; ou
(b) au moins deux types de collagène.

**7.** Dispositif selon l'une quelconque des revendications précédentes, qui comprend en outre un agent favorisant la croissance cellulaire.

**8.** Dispositif selon la revendication 7, dans lequel l'agent favorisant la croissance cellulaire est un peptide ou est choisi parmi des facteurs neurotrophiques, des facteurs de croissance nerveuse et des facteurs de croissance épidermique.

**9.** Dispositif selon la revendication 8, dans lequel le peptide comporte une séquence d'acides aminés de RGD, YIGSR, ou IKVAV.

**10.** Dispositif selon la revendication 8, dans lequel l'agent favorisant la croissance cellulaire est réparti sensiblement dans tout le dispositif.

**11.** Dispositif selon la revendication 1, dans lequel le collagène :

(a) est le seul polymère gonflable à l'eau du dispositif ; et/ou
(b) était réticulé à un pH acide.

**12.** Dispositif selon la revendication 11, dans lequel le pH acide se situe entre 5,0 et 5,5.

**13.** Dispositif selon l'une quelconque des revendications précédentes, dans lequel le collagène réticulé comprend :

(a) de l'atélocollagène, du collagène de type I, du collagène de type III ou une combinaison de ceux-ci ;
(b) un collagène recombinant ; ou
(c) un collagène isolé d'un animal.

**14.** Dispositif selon l'une quelconque des revendications précédentes, dans lequel le collagène réticulé est produit par le mélange ensemble des polymères de collagène et de l'agent de réticulation à un pH acide tout en empêchant des montées de pH.

**15.** Dispositif selon l'une quelconque des revendications précédentes, dans lequel le mélange de polymères et de l'agent de réticulation se situe à un taux de cisaillement élevé.

**16.** Dispositif selon la revendication 1, qui comprend en outre le sulfate de chondroïtine, N,O-carboxyméthylchitosane, l'acide hyaluronique, l'aldéhyde d'acide hyaluronique ou l'alginate.

**17.** Procédé de fabrication d'une composition selon l'une quelconque des revendications 1 à 16, comprenant :

la combinaison de polymères de collagène à l'aide de 1-éthyl-3-(3-diméthylaminopropyl)carbodiimide et d'agents de réticulation de N-hydroxysuccinimide à un pH acide ; et
le durcissement de la combinaison obtenue pour former la composition comprenant le collagène réticulé.

**18.** Procédé selon la revendication 17, dans lequel :

(a) l'étape de combinaison comprend le mélange des polymères de collagène et de l'agent de réticulation dans un système conçu pour produire des forces de cisaillement élevées sur la combinaison obtenue ; et
(b) la combinaison se produit à une température entre 0 °C et 5 °C.

**19.** Procédé selon la revendication 17 ou 18, comprenant en outre l'ajout d'un agent favorisant la croissance cellulaire à la combinaison.

**20.** Dispositif selon l'une quelconque des revendications 1 à 16 destiné à une utilisation dans le traitement d'une maladie, d'un trouble ou d'une blessure ophtalmique chez un sujet en ayant besoin.

**21.** Procédé selon la revendication 20 dans lequel un oeil du sujet présentant ladite maladie, ledit trouble ou ladite blessure ophtalmique est mis en contact avec ledit dispositif ophtalmique.

**22.** Dispositif selon l'une quelconque des revendications 1 à 16 destiné à une utilisation dans la facilitation de la croissance nerveuse sur et/ou dans le dispositif.

**Fig.1.**

**Fig.2**

**Fig.3**

FIG. 8

**Growth of Human Epithelial Cells on Collagen Hyrdogel F1**

FIG. 4

FIG. 7

Growth of Human Epithelial Cells on Collagen Hyrdogel F3

FIG. 5

Growth of Human Epithelial Cells on Collagen Hyrdogel F6

FIG. 6

FIG. 8A

FIG. 9

FIG. 10

FIG. 11

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004014969 A1
- US 5713957 A
- US 5716633 A
- US 5836313 A
- US 6454800 B
- US 6689165 B
- WO 9307889 A
- WO 9416570 A
- WO 2004015090 A
- US 6086204 A, Magnate
- WO 2004028356 A
- US 60573657 B
- US 66140003 A
- US 57365704 P

**Non-patent literature cited in the description**

- *CHEMICAL ABSTRACTS,* 1892-57-5
- The Cornea and Sclera. **MAURICE D M.** The Eye. Academic Press, 1969, vol. I, 489-600
- **DELUSTRO et al.** *J Biomed Mater Res.,* January 1986, vol. 20 (1), 109-20
- **STENZEL et al.** *Annu Rev Biophys Bioeng,* 1974, vol. 3 (0), 231-53
- **LI et al.** *PNAS,* 2003, vol. 100, 15346-15351